# EUROPEAN PATENT SPECIFICATION

(11) **EP 3 613 371 B1**
(45) Date of publication and mention of the grant of the patent: **23.02.2022**
(21) Application number: 18787120.7
(22) Date of filing: 13.04.2018
(51) Int. Cl.: A61B 18/04, A61B 17/32, A61B 18/12, A61B 17/3209, A61B 17/3203, A61B 18/00

(54) **PLASMA IRRADIATION DEVICE, HANDPIECE, AND SURGICAL OPERATION DEVICE**
PLASMASPRITZVORRICHTUNG, HANDSTÜCK UND CHIRURGISCHE OPERATIONSVORRICHTUNG
DISPOSITIF D'IRRADIATION DE PLASMA, PIÈCE À MAIN ET DISPOSITIF D'OPÉRATION CHIRURGICALE

(30) Priority: 19.04.2017 JP 2017082709
(43) Date of publication of application: 26.02.2020
(60) Divisional application: 21186439.2
(73) Proprietor: NGK Spark Plug Co., Ltd., Nagoya-shi, Aichi 467-8525 (JP)
(72) Inventor: ITOH, Shinsuke, Nagoya-shi Aichi 467-8525 (JP); YAMADA, Takaaki, Nagoya-shi Aichi 467-8525 (JP)
(74) Representative: Zimmermann & Partner Patentanwälte mbB
(86) International application number: PCT/JP2018/015589
(87) International publication number: WO 2018/193997

(56) References cited:
- EP-A1- 2 449 992
- EP-A1- 2 591 742
- WO-A1-2012/005132
- WO-A1-2016/071680
- JP-A- 2011 000 224
- JP-A- 2014 519 875
- US-A1- 2006 058 782
- US-A1- 2011 276 113
- US-A1- 2014 074 090
- US-A1- 2016 128 769

## Description

### TECHNICAL FIELD

The present description relates to a plasma irradiation device used in a surgical operation device, to a handpiece, and to the surgical operation device.

### BACKGROUND ART

A technique disclosed in Patent Document 1 has been proposed for a surgical operation device which utilizes plasma. The electric surgical operation device disclosed in Patent Document 1 is configured to produce discharge from an electrode to a tissue through a continuously plasmatized inert gas, thereby incising the tissue and simultaneously causing coagulation.

### PRIOR ART DOCUMENTS

### PATENT DOCUMENTS

Patent Document 1: Japanese Kohyo (PCT) Patent Publication No. 2013-545530
Patent Document 2: Japanese Kohyo (PCT) Patent Publication No. 2013-544122

US 2014/074090 describes a medical device for tissue welding using cold plasma produced by a plasma head.

US 2011/0276113 A1 describes a RF tissue modulation device configured to generate plasma.

### SUMMARY OF THE INVENTION

### PROBLEM TO BE SOLVED BY THE INVENTION

Although the electric surgical operation device disclosed in Patent Document 1 is advantageous in that incision of biological tissue and hemostasis (coagulation) can be performed through use of a single device, the large amount of heat applied to a hemostasis site may result in occurrence of thermal damage, etc. at the hemostasis site.

Meanwhile, Patent Document 2 discloses a hemostasis instrument in which a thermal hemostasis device for tissue coagulation and a biochemical hemostasis device are combined. This hemostasis instrument allows selective use of thermal hemostasis performed through external or internal heating of a tissue and biochemical hemostasis performed through dielectric barrier discharge. When the biochemical hemostasis is chosen, since a non-heating stanching treatment is performed through irradiation with low-temperature plasma, hemostasis is realized while thermal damage to the tissue is suppressed. However, in the biochemical hemostasis device employed in Patent Document 2, a discharge electrode and a tissue function as a pair of electrodes, and dielectric barrier discharge occurs therebetween via a dielectric member. Namely, since the biochemical hemostasis device employs a scheme in which the biological tissue itself is used as an electrode, there is a concern that current may flow into the biological tissue.

The present invention has been accomplished so as to at least partially solve the above-described problem. It is an object of the present invention to provide a handpiece which can perform at least one of incision, ablation, and hemostasis of biological tissue using an acting member and which has a configuration for allowing hemostasis through irradiation with low-temperature plasma and for suppressing heating and energization of the biological tissue during the hemostasis through irradiation with low-temperature plasma.

### MEANS FOR SOLVING THE PROBLEM

The invention is set out in the appended set of claims.

A plasma irradiation device which is a first means for solution is a plasma irradiation device provided in a handpiece including an acting member which acts on biological tissue, comprising:
a gas flow channel for supplying to a distal end portion of the acting member a gas supplied externally of the handpiece; and
an electric field generation section disposed in the gas flow channel, the electric field generation section including a first electrode portion, a second electrode portion facing the first electrode portion, and a dielectric member having at least a portion which is located between the first electrode portion and the second electrode portion and is disposed on at least one of a surface of the first electrode portion and a surface of the second electrode portion, the electric field generation section generating an electric field in a space within the gas flow channel by using a potential difference between the first electrode portion and the second electrode portion, thereby producing the low-temperature plasma discharge.

The plasma irradiation device having the above-described structure can add a function of hemostasis through irradiation with low-temperature plasma to the handpiece which has the basic function of performing at least one of incision, ablation, and hemostasis of biological tissue by using an acting member. Therefore, an operator can perform both the treatments (the treatment using the above-described basic function and the stanching treatment through irradiation with low-temperature plasma) through use of the common handpiece. Since these treatments can be performed by using the common handpiece, the number of instruments used for an operation target can be reduced, whereby the burden on the operation target can be mitigated more easily. In addition, since the stanching treatment is performed by causing coagulation of blood through irradiation with low-temperature plasma, minimally invasive hemostasis is possible. Also, the gas flow channel is configured to supply the gas to a distal end portion of the acting member, and low-temperature plasma is produced within the gas flow channel. Therefore, the low-temperature plasma can be effectively supplied to the vicinity of a region where the actions provided by the above-described basic function (incising action, ablating action, or stanching action) are provided for biological tissue (the vicinity of the distal end portion of the acting member). Further, the first electrode portion and the second electrode portions are disposed in the handpiece, and an electric field based on the potential difference therebetween is produced in the space within the gas flow channel provided in the handpiece. Therefore, low-temperature plasma can be produced in the handpiece without forcedly applying voltage between the operation target and the handpiece or forcedly causing current to flow to the operation target.

In a non-inventive variation, the dielectric member may be configured such that the portion located between the first electrode portion and the second electrode portion is in contact with one of the surface of the first electrode portion and the surface of the second electrode portion. The plasma irradiation device may be configured such that the space within the gas flow channel is present between the first electrode portion and the second electrode portion, and the low-temperature plasma discharge is produced in the space.

This plasma irradiation device can stably generate plasma in the space within the gas flow channel present between the first electrode portion and the second electrode portion. The plasma produced as a result of the discharge can be efficiently supplied to the distal end side of the acting member through use of the flow of the gas in the gas flow channel.

The dielectric member may have a first dielectric member portion disposed on a surface of the second electrode portion on a side toward the first electrode portion and a second dielectric member portion disposed on a surface of the second electrode portion on a side opposite the surface on the side toward the first electrode portion. The second electrode portion may be an electrode whose potential oscillates such that a potential of the first electrode portion becomes the center of the potential oscillation. The second dielectric member portion may have a thickness greater than a thickness of the first dielectric member portion.

In the case where, as described above, the thickness of the second dielectric member portion disposed on the surface of the second electrode portion on the side opposite the surface on the first electrode portion side is greater than the thickness of the first dielectric member portion disposed on the surface of the second electrode portion on the first electrode portion side, even when the potential of the second electrode portion becomes high due to the oscillation of the potential, the influence of the high potential becomes unlikely to reach a region on the outer side of the second dielectric member portion (a region on the side opposite the gas flow channel). As a result, a problem caused by the potential of the second electrode portion becomes less likely to occur in the region on the outer side of the second dielectric member portion. In contrast, the influence of the potential of the second electrode portion becomes more likely to reach the gas flow channel, so that the field intensity can be increased more easily within the gas flow channel.

The acting member may have a rod-like shape, and at least a portion of the acting member may serve as the first electrode portion and serve as a ground electrode.

In the case where the acting member has a rod-like shape and serves as the first electrode portion, the size and the number of components can be reduced more easily. Also, since the acting member is the ground electrode, the potential of a portion which is brought to the close vicinity of biological tissue can be made low. Therefore, even when the acting member is brought to the vicinity of the biological tissue, supply of electricity from the acting member to the biological tissue can be suppressed.

The second electrode portion may be disposed around the first electrode portion in a continuous or intermittent annular pattern. The gas flow channel may be disposed around the first electrode portion to be located on the inner side of the second electrode portion disposed in the annular pattern.

In the case where the gas flow channel is disposed around the first electrode portion to be located on the inner side of the second electrode portion disposed in an annular pattern as described above, electric fields can be produced over the entire circumference of the first electrode portion. As a result, low-temperature plasma can be produced more efficiently in the space within the gas flow channel present around the first electrode portion.

The dielectric member is configured such that the portion located between the first electrode portion and the second electrode portion is in contact with both the surface of the first electrode portion and the surface of the second electrode portion. The dielectric member may be configured such that at least a portion of the dielectric member forms an inner wall portion of the gas flow channel, and the low-temperature plasma discharge is produced along the inner wall portion.

This plasma irradiation device can produce low-temperature plasma discharge along the inner wall portion of the gas flow channel formed by the dielectric member and efficiently supply low-temperature plasma produced as a result of the discharge toward the distal end portion side of the acting member by utilizing the flow of the gas within the gas flow channel. Also, since the low-temperature plasma discharge can be produced in a relatively narrow region along the surfaces of the dielectric members, size reduction is easily realized.

In the above-described configuration in which low-temperature plasma discharge can be produced along the inner wall portion of the gas flow channel, the dielectric member may have a first dielectric member portion disposed on a surface of the second electrode portion on a side toward the first electrode portion and a second dielectric member portion disposed on a surface of the second electrode portion on a side opposite the surface on the side toward the first electrode portion. The second electrode portion may be an electrode whose potential oscillates such that a potential of the first electrode portion becomes the center of the potential oscillation. The second dielectric member portion may have a thickness greater than a thickness of the first dielectric member portion.

In the case where, as described above, the thickness of the second dielectric member portion disposed on the surface of the second electrode portion on the side opposite the surface on the first electrode portion side is greater than the thickness of the first dielectric member portion disposed on the surface of the second electrode portion on the first electrode portion side, even when the potential of the second electrode portion becomes high due to the oscillation of the potential, the influence of the high potential becomes unlikely to reach a region on the outer side of the second dielectric member portion (a region on the side opposite the gas flow channel). As a result, a problem caused by the potential of the second electrode portion becomes less likely to occur in the region on the outer side of the second dielectric member portion. In contrast, the influence of the potential of the second electrode portion becomes more likely to reach the gas flow channel, so that the field intensity can be increased more easily within the gas flow channel.

In the above-described configuration in which low-temperature plasma discharge can be produced along the inner wall portion of the gas flow channel, the first electrode portion may be disposed in a continuous or intermittent annular pattern. The second electrode portion may be disposed in a continuous or intermittent annular pattern around the first electrode portion disposed in the annular pattern.

Since the first electrode portion and the second electrode portion are annularly disposed as described above, a wider region for generation of low-temperature plasma discharge can be secured.

The plasma irradiation device may comprise a tubular portion which includes the acting member provided therein and extending in a predetermined direction. The acting member may have a rod-like shape, and one end portion of the acting member may serve as an acting portion acting on biological tissue.

According to this configuration, the plasma irradiation device can be configured such that the rod-shaped acting member whose one end portion serves as an acting portion (a portion acting on biological tissue) is disposed inside the tubular portion, whereby low-temperature plasma can be supplied toward the acting portion in the plasma irradiation device having such a structure.

The electric field generation section may be provided in the tubular portion to be located at a position corresponding to the one end portion of the acting member (a position corresponding to the acting portion acting on biological tissue.

Since the electric field generation section is configured to produce low-temperature plasma discharge on the one end side of the acting member (the side toward the acting portion which acts on biological tissue) as described above, the low-temperature plasma produced as a result of discharge becomes more likely to be efficiently supplied to the vicinity of the acting portion.

A handpiece which is a second means for solution comprises the above-described plasma irradiation device which is the first means for solution, and a drive section for driving the acting member. The drive section is an ultrasonic vibration section for generating ultrasonic vibration. The acting member vibrates as a result of transmission of the ultrasonic vibration generated by the ultrasonic vibration section to the acting member, thereby performing an incising action, an ablating action, or a thermocoagulation stanching action for the biological tissue.

This handpiece yields effects similar to those of the plasma irradiation device of the first means for solution. Further, this handpiece allows an operator to perform, through use of the common handpiece, incision, ablation, or hemostasis (through thermocoagulation) of biological tissue by ultrasonic vibration, as well as hemostasis through irradiation with low-temperature plasma.

A handpiece which is a third means for solution comprises the above-described plasma irradiation device which is the first means for solution, and a drive section for driving the acting member. The drive section is a high-frequency current supply section for supplying high-frequency current. As a result of the high-frequency current supplied from the high-frequency current supply section flowing through the acting member, the acting member performs an incising action, an ablating action, or a thermocoagulation stanching action for the biological tissue.

This handpiece yields effects similar to those of the plasma irradiation device of the first means for solution. Further, this handpiece allows an operator to perform, through use of the common handpiece, incision, ablation, or hemostasis (through thermocoagulation) of biological tissue by high-frequency current flowing through the acting member, as well as hemostasis by irradiation with low-temperature plasma.

A handpiece which is a fourth means for solution comprises the above-described plasma irradiation device which is the first means for solution, and a displacement device for moving the acting member between a projecting position at which the acting member projects from the tubular portion and a retracted position at which the amount of projection of the acting member is smaller than that at the projecting position.

This handpiece yields effects similar to those of the plasma irradiation device of the first means for solution. Further, when necessary, the acting member can be retracted, and the stanching treatment through application of low-temperature plasma can be performed in a state in which the acting member is located at the retracted position.

Any of the above-described features which can be added to the plasma irradiation device of the first means for solution can be added to the handpieces of the second, third, and fourth means for solution.

A surgical operation device which is a fifth means for solution includes the handpiece of any one of the second through fourth means for solution.

This surgical operation device yields effects similar to those of the plasma irradiation device of the first means for solution. Further, this surgical operation device yields effects similar to those of the handpiece of any one of the second through fourth means for solution.

Any of the above-described features which can be added to the plasma irradiation device of the first means for solution can be added to the surgical operation device of the fifth means for solution.

### EFFECTS OF THE INVENTION

According to the present invention, a handpiece which can perform at least one of incision, ablation, and hemostasis of biological tissue using an acting member makes it possible to perform hemostasis through irradiation with low-temperature plasma and suppress heating and energization of the biological tissue at the time of hemostasis through irradiation with low-temperature plasma.

### BRIEF DESCRIPTION OF THE DRAWINGS

[FIG. 1] Schematic view schematically showing a surgical operation device into which a plasma irradiation device of a first embodiment is incorporated.
[FIG. 2] Schematic sectional view schematically showing the sectional configuration of a cut surface of a non-inventive variation of the plasma irradiation device of the first embodiment along the axial direction thereof.
[FIG. 3] Schematic sectional view schematically showing the sectional configuration of a cut surface of the non-inventive variation of the plasma irradiation device of the first embodiment along a direction perpendicular to the axial direction.
[FIG. 4] Explanatory view for describing a method of manufacturing the non-inventive variation of the plasma irradiation device of the first embodiment.
[FIG. 5] Schematic sectional view schematically showing the sectional configuration of a cut surface of a plasma irradiation device of a second embodiment along the axial direction thereof.
[FIG. 6] Schematic sectional view schematically showing the sectional configuration of a cut surface of the plasma irradiation device of the second embodiment along a direction perpendicular to the axial direction.
[FIG. 7] Explanatory view for describing a method of manufacturing the plasma irradiation device of the second embodiment.
[FIG. 8] Schematic view schematically showing a surgical operation device into which a plasma irradiation device of a third embodiment is incorporated.
[FIG. 9] Schematic sectional view schematically showing the sectional configuration of a cut surface of a plasma irradiation device of a fourth embodiment along the axial direction thereof.
[FIG. 10] Schematic view schematically showing a surgical operation device into which a plasma irradiation device of a fifth embodiment is incorporated.
[FIG. 11] Schematic sectional view schematically showing the sectional configuration of a cut surface of a further non-inventive variation of a plasma irradiation device of the fifth embodiment along a direction perpendicular to the axial direction thereof.
[FIG. 12] Schematic view schematically showing a surgical operation device into which a plasma irradiation device of a sixth embodiment is incorporated.
[FIG. 13] Schematic sectional view schematically showing the sectional configuration of a cut surface of the plasma irradiation device of the sixth embodiment along a direction perpendicular to the axial direction thereof.
[FIG. 14] Schematic sectional view schematically showing the sectional configuration of a cut surface of a further non-inventive variation of a plasma irradiation device of another embodiment along a direction perpendicular to the axial direction thereof.

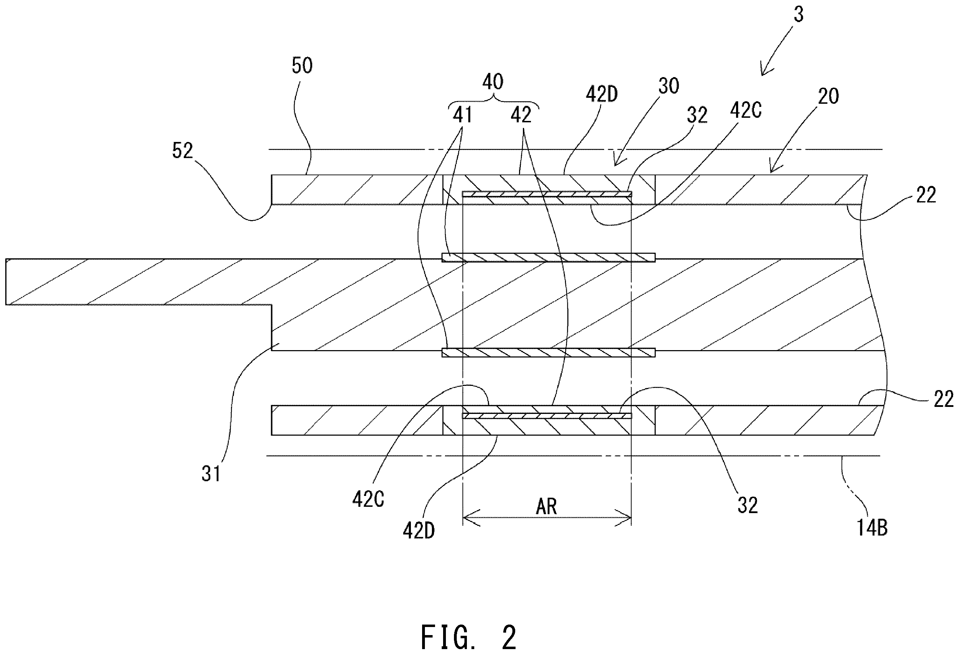

The embodiments shown in Figures 2-4, 11 and 14 are not-inventive as they do not show the feature "dielectric member is configured such that the portion located between the first electrode portion and the second electrode portion is in contact with both the surface of the first electrode portion and the surface of the second electrode portion." However, the description of these embodiments also includes explanations and other features and modifications useful for understanding the general concept of the invention as defined in the appended claims.

### MODES FOR CARRYING OUT THE INVENTION

### <First embodiment, not covered by the claimed invention>

### 1. Overall structure of surgical operation device

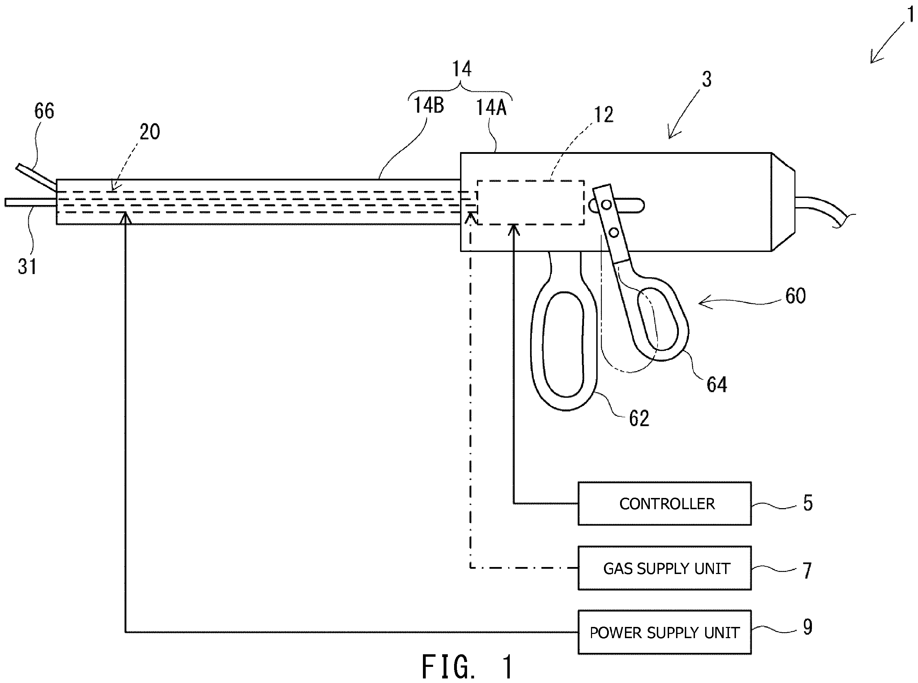

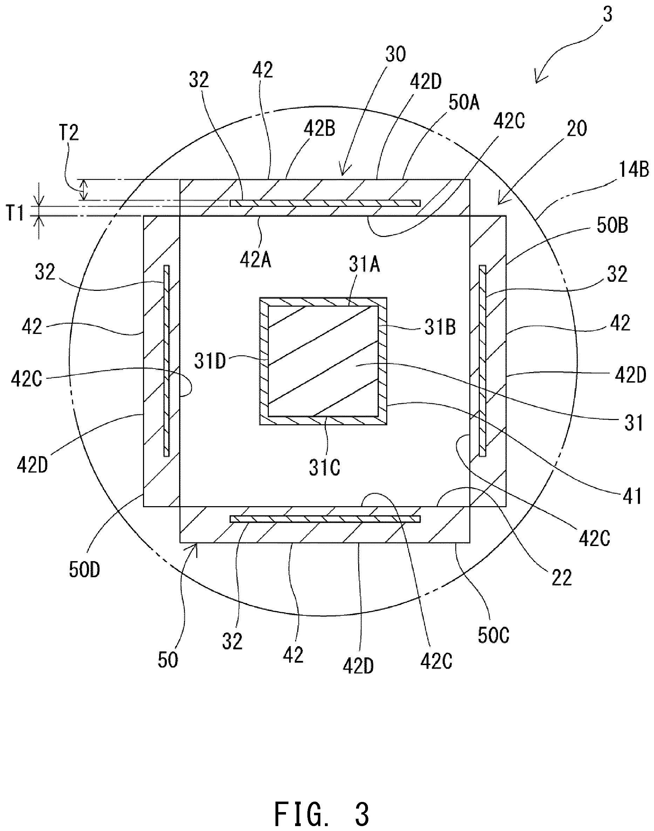

A surgical operation device 1 shown in FIG. 1 is configured as a treatment device for performing incision, ablation, or hemostasis for the biological tissue of an operation target. The surgical operation device 1 mainly includes a handpiece 3; a controller 5 which is an apparatus for controlling an ultrasonic vibration section 12 (drive section); a gas supply unit 7 for supplying a gas to a gas flow channel 22 (FIG. 3) within the handpiece 3; and a power supply unit 9 which can apply voltage to a plasma irradiation device 20.

The controller 5 is an apparatus for providing the ultrasonic vibration section 12 with an electric signal for generation of ultrasonic vibration. The controller 5 is configured to provide the electric signal to the ultrasonic vibration section 12 through, for example, an unillustrated flexible signal cable extending between the handpiece 3 and the controller 5.

The gas supply unit 7 is an apparatus for supplying an inert gas such as helium gas or argon gas. The gas supply unit 7 supplies the inert gas to the gas flow channel 22 through, for example, an unillustrated flexible pipe extending between the handpiece 3 and the gas supply unit 7.

The power supply unit 9 is an apparatus for applying a desired voltage to a first electrode portion (an acting member 31) and second electrode portions 32 of the plasma irradiation device 20, which will be described later. Specifically, the power supply unit 9 supplies an AC voltage of a predetermined frequency between the first electrode portion (the acting member 31) and the second electrode portions 32 while maintaining the acting member 31 (the first electrode portion) at a ground potential. The power supply unit 9 may employ any of various known circuits which can produce AC voltage. Notably, in the example of FIG. 1, there is exemplified a surgical operation device in which the power supply unit 9 for producing the AC voltage applied between the first electrode portion (the acting member 31) and the second electrode portions 32 is provided externally of the handpiece 3. However, a power supply circuit which produces the AC voltage applied between the first electrode portion (the acting member 31) and the second electrode portions 32 may be provided inside the handpiece 3.

The handpiece 3 is an apparatus which is held and used by an operator who performs surgical operation. The handpiece 3 mainly includes the plasma irradiation device 20, the ultrasonic vibration section 12, a movable member displacement mechanism 60, a casing 14, etc.

The casing 14 is composed of a cylindrical first casing 14A extending in a predetermined direction and a cylindrical second casing 14B connected to one end of the first casing 14A and extending in the predetermined direction. The ultrasonic vibration section 12, etc. are accommodated in the first casing 14A, and the plasma irradiation device 20, etc. are accommodated in the second casing 14B. Notably, in FIGS. 2 and 3, the second casing 14B is imaginarily shown by an alternate long and two short dashes line(s). In the present configuration, the first casing 14A corresponds to an example of the proximal portion and is a portion where a grip portion (specifically, a stationary grip portion 62 and a movable grip portion 64) is provided. Also, the second casing 14B corresponds to an example of the extension portion and extends from the first casing 14A (the proximal portion) in the predetermined direction. When the second casing 14B (the extension portion) is configured to have the shape of a cylinder having an outer diameter of, for example, 10 mm or less, the hand piece 3 can be preferably used for endoscopic surgery or the like.

The ultrasonic vibration section 12 is configured as a known ultrasonic vibrator. When a predetermined electric signal is applied to the ultrasonic vibration section 12 by the above-described controller 5, the ultrasonic vibration section 12 operates to transmit an ultrasonic vibration to the acting member 31 having a rod-like shape. This ultrasonic vibration section 12 corresponds to one example of the drive section and drives the acting member 31 in such a manner that an action of incising, ablating, or stanching, through thermocoagulation, biological tissue occurs near a distal end of the acting member 31.

The acting member 31 is a member whose distal end portion acts on the biological tissue as a stationary blade. The acting member 31 corresponds to an example of the vibration member to which the ultrasonic vibration generated by the ultrasonic vibration section 12 is transmitted. When the ultrasonic vibration is transmitted by the ultrasonic vibration section 12, the acting member 31 operates in such a manner that an action of incising, ablating, or stanching, through thermocoagulation, biological tissue occurs near the distal end of the acting member 31. Notably, the acting member 31 will be described later in more detail.

The movable member displacement mechanism 60 is a mechanism which displaces a movable member 66 functioning as a movable blade, and a known moving mechanism is employed. This movable member displacement mechanism 60 includes the stationary grip portion 62 fixed to the first casing 14A; a movable grip portion 64 attached to be movable relative to the stationary grip portion 62; the movable member 66 which moves together with the movable grip portion 64 in an interlocked manner; and an unillustrated interlocking mechanism which is linked to the movable grip portion 64 and the movable member 66 and which moves the movable member 66 in accordance with displacement of the movable grip portion 64. In the movable member displacement mechanism 60, the rod-shaped movable member 66 is pivotable on a pivot axis near a distal end of the second casing 14B. When an operation of moving the movable grip portion 64 toward the stationary grip portion 62 is performed as shown by an alternate long and two short dashes line in FIG. 1, the movable member 66 pivots in such a manner that a distal end portion of the movable member 66 moves toward a distal end portion of the acting member 31. In contrast, when an operation of separating the movable grip portion 64 from the stationary grip portion 62 is performed, the movable member 66 pivots in such a manner that the distal end portion of the movable member 66 moves away from the distal end portion of the acting member 31.

The handpiece 3 configured as described above allows an operator to perform an incising treatment an ablating treatment, and a stanching treatment for biological tissue through use of ultrasonic vibration. For example, when the biological tissue is nipped between the distal end portion of the acting member 31 functioning as a stationary blade and the movable member 66 functioning as a movable blade, a portion of the biological tissue can be cut and removed by the ultrasonic vibration applied to the acting member 31. Also, the acting member 31 to which ultrasonic vibration is applied can be brought into contact with the biological tissue so as to generate frictional heat, thereby performing hemostasis. Also, the ablation treatment can be performed by nipping the biological tissue between the acting member 31 and the movable member 66 with or without application of ultrasonic vibration to the acting member 31. As described above, the handpiece 3 allows the operator to perform incision, ablation, or hemostasis (through thermocoagulation) by using ultrasonic vibration. Further, the handpiece 3 allows the operator to perform minimally invasive hemostasis through application of low-temperature plasma from the plasma irradiation device 20 which will be described later. This minimally invasive hemostasis can be performed in a state in which the biological tissue is nipped between the distal end portion of the acting member 31 functioning as a stationary blade and the movable member 66 functioning as a movable blade.

### 2. Structure of plasma irradiation device

Next, the structure of the plasma irradiation device 20 will be described in detail.

As shown in FIG. 1, the plasma irradiation device 20 is incorporated into the handpiece 3 and is configured as an apparatus which produces dielectric barrier discharge inside the handpiece 3. As shown in FIGS. 2 and 3, the plasma irradiation device 20 mainly includes a gas flow channel 22 and an electric field generation section 30.

As shown in FIG. 3, the gas flow channel 22 is provided in such a manner that a portion of the acting member 31 constituting the handpiece 3 is inserted into the gas flow channel 22. The gas flow channel 22 serves as a channel through which the inert gas supplied from the outside (specifically, the gas supply unit 7) of the handpiece 3 flows toward the distal end portion of the acting member 31.

As shown in FIG. 2, the plasma irradiation device 20 includes a tubular portion 50 which has a tubular shape and extends in a predetermined direction (the extending direction of the second casing 14B). The tubular portion 50 is provided in such a manner that the tubular portion 50 is accommodated in the second casing 14B shown in FIG. 1. As shown in FIG. 2, the acting member 31 is inserted into the tubular portion 50 in such a manner that the acting member 31 is disposed at the center of the tubular portion 50. The acting member 31 is a square-rod-shaped shaft member. In an axially extending region AR where the second electrode portions 32 are provided, as shown in FIG. 3, a cross section of the acting member 31 taken perpendicular to the axial direction has a rectangular (specifically, square) outer edge shape. The gas flow channel 22 is defined by an inner surface portion (inner wall portion) of the tubular portion 50 and an outer surface portion of the acting member 31 (the shaft member). The inert gas flows through the space between the tubular portion 50 and the acting member 31 from the proximal end side of the second casing 14B (the first casing 14A side) toward the distal end side thereof.

As shown in FIG. 2, the electric field generation section 30 has a first electrode portion (the acting member 31), second electrode portions 32, and a dielectric member 40 interposed between the first electrode portion (the acting member 31) and the second electrode portions 32. The electric field generation section 30 is incorporated into the handpiece 3 (FIG. 1). The electric field generation section 30 functions to generate an electric field within the gas flow channel 22 by using a potential difference between the first electrode portion (the acting member 31) and the second electrode portions 32, thereby producing low-temperature plasma discharge. The electric field generation section 30 operates to produce low-temperature plasma in the elongated handpiece 3 at a position near one end thereof in the longitudinal direction (a position near the distal end of the acting member 31). Specifically, the electric field generation section 30 produces low-temperature plasma at a position near the distal end of the second casing 14B (the extension portion) (a position on the distal end side of the longitudinal center of the second casing 14B).

As shown in FIG. 3, the dielectric member 40 includes a first-electrode-portion-side dielectric member 41 which covers the acting member 31 (the first electrode portion) and second-electrode-portion-side dielectric members 42 in which the second electrode portions 32 are embedded. For example, a ceramic material such as alumina or a glass material can be preferably used as the materials of the first-electrode-portion-side dielectric member 41 and the second-electrode-portion-side dielectric members 42. Notably, when alumina which is high in mechanical strength is used as a dielectric material, the size of the electric field generation section 30 can be easily reduced.

As shown in FIGS. 2 and 3, the first-electrode-portion-side dielectric member 41 is disposed to annularly surround the acting member 31 in a predetermined region in the axial direction of the acting member 31. Specifically, as shown in FIG. 2, the first-electrode-portion-side dielectric member 41 surrounds the acting member 31 over a region wider than the region AR as viewed in the axial direction of the acting member 31 such that the first-electrode-portion-side dielectric member 41 extends over the entire region AR where the second electrode portions 32 are disposed and the first-electrode-portion-side dielectric member 41 is present on axially opposite sides of the region AR. The first-electrode-portion-side dielectric member 41 is formed such that the acting member 31 is not exposed to the gas flow channel 22 at least in the region AR in the axial direction of the acting member 31. Notably, a recess having a shape corresponding to the shape of the first-electrode-portion-side dielectric member 41 may be formed on the surface of the acting member 31, and the first-electrode-portion-side dielectric member 41 may be fitted into the recess. Since such a structure can reduce the numbers of recesses and projections of the surface of the gas flow channel 22, disturbance of the flowing inert gas can be prevented, and low-temperature plasma can be produced stably.

As shown in FIG. 2, the acting member 31 functions as a rod-shaped electrode member portion serving as a first electrode portion and also serving as a ground electrode. As shown in FIG. 3, the acting member 31 has the shape of a square rod whose cross section has a rectangular (specifically, square) outer edge shape over at least the entire axial region where the second electrode portions 32 and the first-electrode-portion-side dielectric member 41 are provided. Meanwhile, the second electrode portions 32 are disposed around the acting member 31 (the rod-shaped electrode member) in an intermittent annular pattern. The gas flow channel 22 is formed around the acting member 31 (the rod-shaped electrode member) to be located on the inner side of the second electrode portions 32 disposed in the annular pattern.

As shown in FIGS. 2 and 3, the second electrode portions 32 are embedded in the second-electrode-portion-side dielectric members 42, so that the second electrode portions 32 are not exposed to the gas flow channel 22. In the region where the second electrode portions 32 are provided, the inner wall of the gas flow channel 22 is formed by the second-electrode-portion-side dielectric members 42. The second electrode portions 32 have a layered structure and are disposed to face respective outer surface portions 31A, 31B, 31C, and 31D of the acting member 31. In the example of FIG. 3, the second electrode portions 32 are disposed in the plurality of second-electrode-portion-side dielectric members 42 each having a plate-like shape, whereby respective wall portions 50A, 50B, 50C, and 50D of the tubular portion 50 are formed. The plurality of wall portions 50A, 50B, 50C, and 50D have the same structure and are connected in an annular shape, whereby the tubular portion 50 is formed. Each of the second-electrode-portion-side dielectric members 42 of the plurality of wall portions 50A, 50B, 50C, and 50D has a first dielectric member portion 42C disposed on a surface of the corresponding second electrode portion 32 on the acting member 31 side (on the first electrode portion side) and a second dielectric member portion 42D disposed on a surface of the corresponding second electrode portion 32 on the side opposite the surface on the acting member 31 side. The thickness T2 of the second dielectric member portion 42D is greater than the thickness T1 of the first dielectric member portion 42C. The thickness T1 is equal to the spacing between the second electrode portion 32 and the surface 42A of the second-electrode-portion-side dielectric member 42 on the side toward the gas flow channel 22. The thickness T2 is equal to the spacing between the second electrode portion 32 and the surface 42B of the second-electrode-portion-side dielectric member 42 on the side opposite the gas flow channel 22.

In the plasma irradiation device 20 configured as described above, the space of the gas flow channel 22 is present between the acting member 31 (the first electrode portion) and the second electrode portions 32, and the inert gas flows through the space. The power supply unit 9 applies an AC voltage having a predetermined frequency between the acting member 31 and the second electrode portions 32. This power supply unit 9 operates to maintain the acting member 31 (the first electrode portion) at the ground potential and oscillationally change the potentials of the second electrode portions 32 within a range between a potential of +A (V) and a potential of -A (V). The potential of +A (V) is higher than the potential of the acting member 31 (which is the ground potential and is the center of the range) by a predetermined amount. The potential of -A (V) is lower than the potential of the acting member 31 by a predetermined amount. Notably, "A" is a positive value. When the AC voltage is applied, changes in electric field occur between the acting member 31 and the second electrode portions 32 in a state in which respective barriers are formed on the acting member 31 and the second electrode portions 32 by the dielectric member 40. As a result, dielectric barrier discharge is produced in the space within the gas flow channel 22 present between the acting member 31 (the first electrode portion) and the second electrode portions 32. In the gas flow channel 22, the inert gas flows toward an end portion of the gas flow channel 22 (specifically, an opening portion 52 forming an end portion of the tubular portion 50 shown in FIG. 2). The low-temperature plasma produced as a result of the dielectric barrier discharge is discharged from the end portion of the gas flow channel 22 toward the distal end side of the acting member 31. Since the handpiece 3 has the structure as described above, the operator can irradiate a bleeding portion with the low-temperature plasma by operating the handpiece 3 such that its distal end portion (a distal end portion of the acting member 31) is directed toward the bleeding portion and by activating the plasma irradiation device 20. As a result, blood coagulation occurs, whereby a stanching treatment can be performed.

### 3. Method of manufacturing plasma irradiation device, not covered by the claimed invention

Next, a method of manufacturing the plasma irradiation device 20 will be described. Here, a method of manufacturing the wall portions (wall portions 50A, 50B, 50C, and 50D) constituting the tubular portion 50 shown in FIG. 3 will be mainly described.

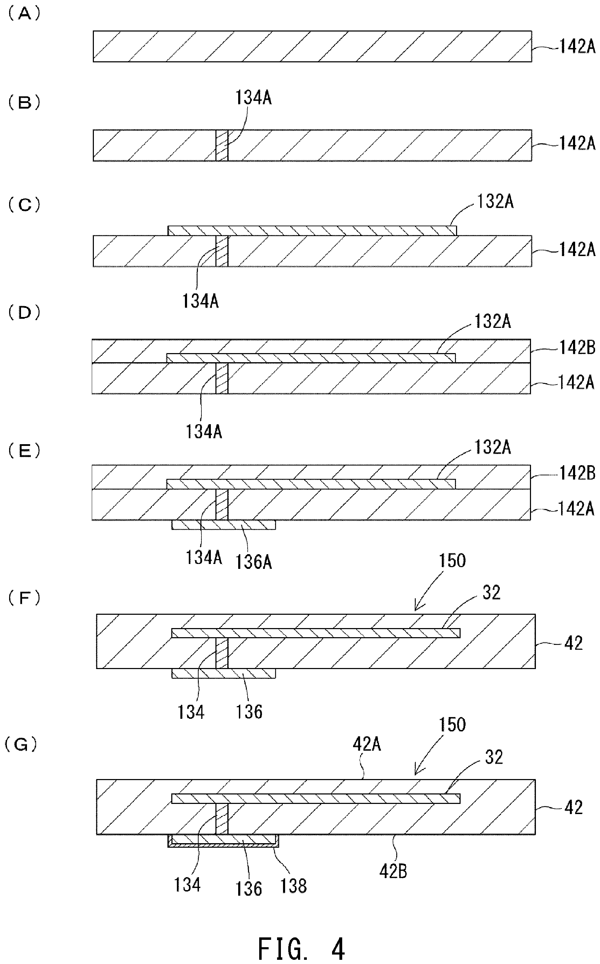

For manufacture of the wall portions constituting the tubular portion 50, a first ceramic green sheet formation step is performed first. As shown in FIG. 4(A), in the first ceramic green sheet formation step, a first ceramic green sheet 142A having a predetermined thickness is formed by using a ceramic material containing alumina powder as a main component. Here, any of known methods such as tape molding and extrusion molding may be used as a method of forming the ceramic green sheet. After the first ceramic green sheet formation step, a via hole formation step is performed. In the via hole formation step, laser machining is first performed on the first ceramic green sheet formed as shown in FIG. 4(A), whereby a through hole for a via hole 134 (see FIG. 4(G)) is formed. Notably, for formation of the through hole, any of known methods such as punching and drilling may be used. Next, an electrically conductive paste (tungsten paste in the present embodiment) is charged into the through hole for the via hole 134 by using a known paste printing device (not shown), whereby an unfired via hole conductor portion 134A which is to become a conductor of the via hole 134 is formed. Notably, FIG. 4(B) conceptually shows a state in which the unfired green via hole conductor portion 134A is formed in the first ceramic green sheet 142A.

After having performed the first ceramic green sheet formation step as shown in FIG. 4(A) and the via hole formation step as shown in FIG. 4(B), an unfired conductive layer formation step is performed. In the unfired conductive layer formation step, as shown in FIG. 4(C), a tungsten paste 132A containing tungsten as a main component is applied (printed) on one side of the first ceramic green sheet 142A by using a known paste printing device (not shown).

After the unfired conductive layer formation step, a second ceramic green sheet formation step is performed. In the second ceramic green sheet formation step, a second ceramic green sheet 142B having a predetermined thickness is formed by using a ceramic material containing alumina powder as a main component, and, as shown in FIG. 4(D), this second ceramic green sheet 142B is placed on a laminate obtained in the unfired conductive layer formation step (a laminate composed of the first ceramic green sheet 142A and the tungsten paste 132A), and a pressing force is applied in the sheet stacking direction for pressure bonding, whereby the ceramic green sheet 142B is integrated with the laminate. Further, as shown in FIG. 4(E), a surface electrode portion formation step is performed. In the surface electrode portion formation step, by using a known paste printing device (not shown), an electrically conductive paste is printed on a main face of the first ceramic green sheet 142A, on which main face the unfired via hole conductor portion 134A has been formed, whereby an unfired surface electrode portion 136A is formed. The unfired surface electrode portion 136A is a portion which will become a surface electrode portion 136 (see FIG. 4(G)) after firing.

Next, after performing a drying step and a debindering step in accordance with known procedures, a firing step of heating the ceramic laminate (ceramic green sheets and unfired electrodes) to a predetermined temperature (for example, about 1400°C to 1600°C) at which alumina and tungsten can sinter is performed. As a result of this firing step, as shown in FIG. 4(F), alumina in the ceramic green sheets and tungsten in the tungsten paste sinter, whereby a plate-shaped member 150 (a plate-shaped member in which a second electrode portion 32 and a via hole 134 are embedded in a dielectric member 42 and a surface electrode portion 136 is formed on the dielectric member 42) is produced.

A plating layer 138 (for example, an Ni plating layer) is formed on the plate-shaped member 150 produced as described above such that the plating layer 138 covers the surface electrode portion 136. In this structure, the second electrode portion 32 electrically communicates with the surface electrode portion 136 through the via hole 134. Accordingly, it becomes possible to set the potential of the second electrode portion 32 through the surface electrode portion 136.

A plurality of plate-shaped members 150 (FIG. 4(G)) which are to become the wall portions of the tubular portion 50 are formed through the above-described steps and are disposed annularly, whereby at least a portion of the tubular portion 50 (an annular portion in which the second electrode portions 32 are embedded as shown in FIG. 3) can be formed. Each of the wall portions 50A, 50B, 50C, and 50D shown in FIG. 3 is formed by the plate-shaped member 150 as shown in FIG. 4(G). Notably, in FIG. 3, the surface electrode portions 136, the via holes 134, etc. are omitted. The tubular portion 50 may be formed into a square tubular shape through use of only the plurality of plate-shaped members 150. Alternatively, the tubular portion 50 may be formed by connecting another annular member (for example, a square tubular member made of an insulating material which is the same as or different from the material of the dielectric member 40) to the plurality of plate-shaped members 150 forming a squire tubular shape.

After formation of the tubular portion 50, the acting member 31 (specifically, a rod-shaped body which includes a shaft member made of metal and machined into a predetermined shape and a dielectric member (the first-electrode-portion-side dielectric member 41) covering a portion of the shaft member) is disposed inside the tubular portion 50, and the tubular portion 50 and the acting member 31 are held such that they have a predetermined positional relation therebetween, whereby the plasma irradiation device 20 shown in FIG. 2 and 3 is obtained.

Notably, in the example of FIG. 4, the surface electrode portion 136 is disposed on the surface 42B of the plate-shaped member 150, which surface is located on the side opposite the surface 42A which is to become the inner wall surface of the gas flow channel, whereby electrical communication between the second electrode portion 32 and an outside circuit is established. However, the second electrode portion 32 may be exposed to the outside on an end surface (side wall surface) of the plate-shaped member 150, and an electrode portion may be disposed on the end surface so as to establish electrical communication between the second electrode portion 32 and the outside circuit.

Next, the effects of the present configuration will be described.

The plasma irradiation device 20 can add a function of hemostasis through irradiation with low-temperature plasma to the handpiece 3 which has a basic function of incising or ablating biological tissue or hemostasis through thermocoagulation. Therefore, an operator can perform both the treatments (the treatment using the above-described basic function and the stanching treatment through irradiation with low-temperature plasma) through use of the common handpiece 3. Since these treatments can be performed by using the common handpiece 3, the number of instruments used for an operation target can be reduced, whereby the burden on the operation target can be mitigated more easily.

In an assumed case where the handpiece 3 is applied to, for example, endoscopic surgery, since the plasma irradiation device 20 is integrated with a device which can perform incision, ablation, or hemostasis (through thermocoagulation) by using ultrasonic vibration, the number of instruments inserted into the abdominal cavity can be reduced, whereby the burden on a patient can be reduced further.

In addition, since the stanching treatment is performed by causing coagulation of blood through irradiation with low-temperature plasma, minimally invasive hemostasis is possible.

In particular, since hemostasis through application of low-temperature plasma does not cause thermal damage, the risk of postoperative troubles can be reduced. Also, since a scar stemming from the thermal damage is unlikely to remain, there is a merit that the lesion site can be easily specified at the time of re-operation. Also, since smoke due to application of heat during the stanching treatment is not produced, a problem that the field of view is narrowed by smoke during an operation is unlikely to occur.

Also, the gas flow channel 22 is configured to supply the gas to a distal end portion of the acting member 31, and low-temperature plasma is produced within the gas flow channel 22. Therefore, the low-temperature plasma can be effectively supplied to the vicinity of a region where the actions provided by the above-described basic function (incising action, ablating action, or thermocoagulation stanching action) are provided for biological tissue (the vicinity of the distal end portion of the acting member 31).

Further, the first electrode portion (the acting member 31) and the second electrode portions 32 are disposed in the handpiece 3, and an electric field based on the potential difference therebetween is produced in the space within the gas flow channel 22 provided in the handpiece 3. Therefore, low-temperature plasma can be produced in the handpiece 3 without forcedly applying voltage between the operation target and the handpiece 3 or forcedly causing current to flow to the operation target.

When the ultrasonic vibration generated at the ultrasonic vibration section 12 (the drive section) is transmitted to the acting member 31, the acting member 31 itself vibrates and provides the incising action, the ablating action, or the thermocoagulation stanching action for the biological tissue. By virtue of this configuration, the plasma irradiation device 20 allows an operator to perform, through use of the common handpiece, incision, ablation, or hemostasis (through thermocoagulation) of biological tissue by ultrasonic vibration, as well as minimally invasive hemostasis by irradiation with low-temperature plasma.

The dielectric member 40 is configured such that its portion located between the acting member 31 (the first electrode portion) and the second electrode portions 32 is in contact with only one of the surface of the acting member 31 (the first electrode portion) and the surfaces of the second electrode portions 32 (specifically, only the surfaces of the second electrode portions 32). The plasma irradiation device 20 is configured such that the space within the gas flow channel 22 is present between the acting member 31 (the first electrode portion) and the second electrode portions 32, and low-temperature plasma discharge is produced in the space. This plasma irradiation device 20 can stably generate plasma in the space within the gas flow channel 22 present between the acting member 31 (the first electrode portion) and the second electrode portions 32. The low-temperature plasma produced as a result of the discharge can be efficiently supplied to the distal end side of the acting member 31 through use of the flow of the gas produced in the gas flow channel 22.

The dielectric member 40 includes the first dielectric member portions 42C disposed on the surfaces of the second electrode portions 32 on the side toward the acting member 31 (the first electrode portion), and the second dielectric member portions 42D disposed on the surfaces of the second electrode portions 32 opposite the side toward the acting member 31 (the first electrode portion). Each of the second electrode portions 32 serves as an electrode whose potential oscillates such that the potential of the acting member 31 (the first electrode portion) (the ground potential) becomes the center of the potential oscillation. The thickness T2 of the second dielectric member portions 42D is greater than the thickness T1 of the first dielectric member portions 42C. In the case where the thickness T2 of the second dielectric member portions 42D is greater than the thickness T1 of the first dielectric member portions 42C as described above, even when the potential of the second electrode portions 32 becomes high due to the oscillation of the potential, the influence of the high potential becomes unlikely to reach a region on the outer side of the second dielectric member portions 42D (a region on the side opposite the gas flow channel 22). As a result, a problem caused by the potential of the second electrode portions 32 becomes less likely to occur in the region on the outer side of the second dielectric member portions 42D (for example, on the outer side of the second casing 14B). In contrast, the influence of the potential of the second electrode portions 32 becomes more likely to reach the gas flow channel 22, so that the field intensity can be increased more easily within the gas flow channel 22.

The acting member 31 has a rod-like shape, and at least a portion of the acting member 31 serves as a first electrode portion and a ground electrode. In the case where the acting member 31 has a rod-like shape and also serves as the first electrode portion, the size and the number of components can be reduced more easily. Also, since the acting member 31 is the ground electrode, the potential of a portion which is brought to the close vicinity of biological tissue can be made low. Therefore, even when the acting member 31 is brought to the vicinity of the biological tissue, supply of electricity from the acting member 31 to the biological tissue can be suppressed. Notably, although no limitation is imposed on the potential state of the biological tissue, the biological tissue and the acting member 31 may be maintained at the same ground potential by electrically connecting the acting member 31 (which is formed as a ground electrode) and the biological tissue through an unillustrated wire. In this case, an earth wire for grounding may be electrically connected to the acting member 31 or the biological tissue so that the acting member 31 or the biological tissue are grounded.

The second electrode portions 32 are disposed around the acting member 31 (the first electrode portion) in an intermittent annular pattern. The gas flow channel 22 is disposed around the acting member 31 (the first electrode portion) to be located on the inner side of the second electrode portions 32 disposed in the annular pattern. In the case where, as described above, the gas flow channel 22 is disposed around the acting member 31 (the first electrode portion) to be located on the inner side of the annularly disposed second electrode portions 32, electric fields can be produced over the entire circumference of the acting member 31 (the first electrode portion). As a result, low-temperature plasma can be produced more efficiently in the space within the gas flow channel 22 present around the acting member 31 (the first electrode portion).

The acting member 31 is formed as a vibration member to which the ultrasonic vibration generated by the ultrasonic vibration section 12 is transmitted. The handpiece 3 is configured to provide an incising action, an ablating action, or a thermocoagulation stanching action for biological tissue by utilizing the vibration of the acting member 31 (the vibration member). In this configuration, the acting member 31 serving as the first electrode portion is also used as a vibration member which is brought into contact with biological tissue so as to provide an incising action, an ablating action, or a thermocoagulation stanching action for the biological tissue. Therefore, the number of components can be reduced further, which is advantageous for size reduction. In addition, since the vibration member (the acting member 31) which comes into contact with biological tissue can be stably maintained at the ground potential, the biological tissue is less likely to receive an electrical adverse effect.

The plasma irradiation device 20 has the tubular portion 50 which accommodates the acting member 31 and extends in a predetermined direction (the extension direction of the second casing 14B (the extension portion)). The acting member 31 is formed into a rod-like shape and its portion on one end side thereof serves as an acting portion which acts on biological tissue. By virtue of this configuration, the plasma irradiation device 20 can be configured such that the rod-shaped acting member 31 whose one end portion serves as an acting portion (a portion acting on biological tissue) is disposed inside the tubular portion 50, whereby low-temperature plasma can be supplied toward the acting portion in the plasma irradiation device 20 having such a structure. Specifically, the gas flow channel 22 is formed by an inner wall portion of the tubular portion 50 and an outer surface portion of the acting member 31 or a covering portion (the first-electrode-portion-side dielectric member 41) covering the outer surface portion. In the case where the gas flow channel 22 is formed as described above, it is possible to cause a gas to flow along the acting member 31 in the vicinity of the acting member 31. As a result, low-temperature plasma can be efficiently supplied toward the acting portion (a distal end portion of the acting member 31).

The electric field generation section 30 is provided in the tubular portion 50 to be located at a position corresponding to one end of the acting member 31 (a position corresponding to the acting portion which acts on biological tissue). Since the electric field generation section 30 is configured to produce low-temperature plasma discharge on the one end side of the acting member 31 (the side toward the acting portion which acts on biological tissue), the low-temperature plasma produced as a result of discharge becomes more likely to be efficiently supplied to the vicinity of the acting portion. Specifically, the handpiece 3 having the plasma irradiation device 20 incorporated thereinto includes the first casing 14A (the proximal portion) where the grip portion (the stationary grip portion 62 and the movable grip portion 64) is provided, and the second casing 14B (the extension portion) extending in the predetermined direction from the first casing 14A (the proximal portion). The electric field generation section 30 is provided in the second casing 14B (the extension portion) and is configured to produce low-temperature plasma discharge at a position near the distal end of the second casing 14B (the extension portion). Since the electric field generation section 30 is configured as described above, the low-temperature plasma generated as a result of discharge becomes more likely to be efficiently supplied toward the distal end side of the second casing 14B (the extension portion).

### <Second embodiment, covered by the claimed invention>

Next, a second embodiment will be described.

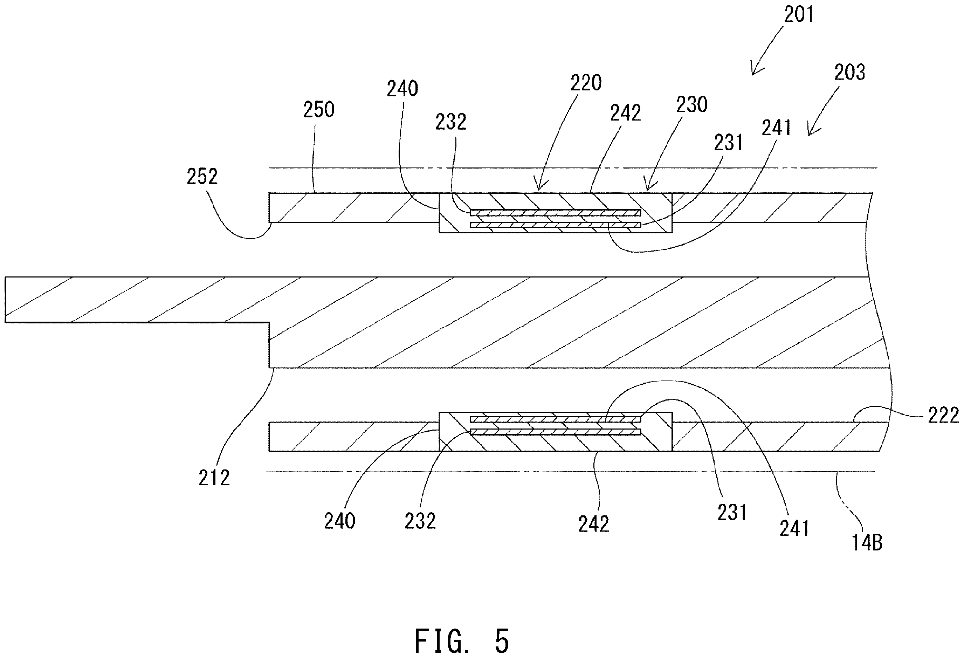

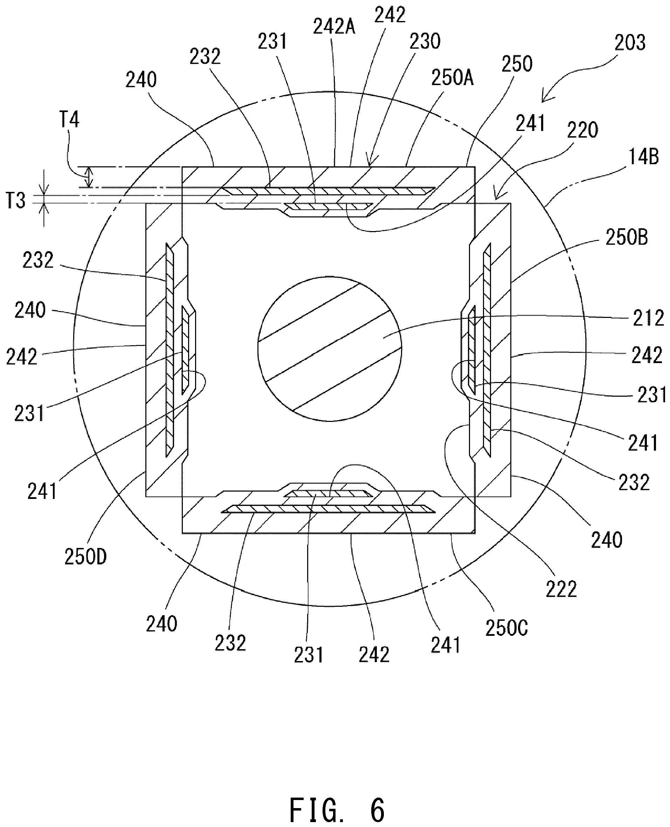

A surgical operation device 201 to which a plasma irradiation device 220 of a second embodiment is applied is obtained by replacing the plasma irradiation device 20 of the surgical operation device 1 shown in FIG. 1 with the plasma irradiation device 220. The structure of the surgical operation device 201 is identical with that of the surgical operation device 1 shown in FIG. 1, except for the plasma irradiation device 220 (which corresponds to the plasma irradiation device 20 in the surgical operation device 1). A handpiece 203 conceptually shown in FIGS. 5 and 6 is identical with the handpiece 3 shown in FIG. 1 except that the plasma irradiation device 20 is replaced with the plasma irradiation device 220. Notably, in the surgical operation device 201 to which the plasma irradiation device 220 of the second embodiment is applied, portions identical with those of the surgical operation device 1 shown in FIG. 1 are denoted by the same symbols as those of the corresponding portions of the surgical operation device 1, and their detailed descriptions will be omitted. Notably, an acting member 212 of the plasma irradiation device 220 functions in the same manner as the acting member 31 of the plasma irradiation device 20 shown by FIG. 1, etc., and serves as the vibration member to which ultrasonic vibration is applied by an ultrasonic vibration section similar to the ultrasonic vibration section 12 shown in FIG. 1. Also, in this example as well, a distal end portion of the acting member 212 serves as an acting portion which acts on biological tissue.

The plasma irradiation device 220 is incorporated into the handpiece 203 and is configured as an apparatus which produces creeping discharge inside the handpiece 203. As shown in FIGS. 5 and 6, the plasma irradiation device 220 mainly includes a gas flow channel 222 and an electric field generation section 230. The gas flow channel 222 is provided in the handpiece 203 and serves as a channel through which the inert gas supplied from the outside of the handpiece 203 (specifically, from an external apparatus similar to the gas supply unit 7 of FIG. 1) flows toward a distal end portion of the acting member 31.

As shown in FIGS. 5 and 6, the plasma irradiation device 220 includes a tubular portion 250 which has a tubular shape and extends in a predetermined direction (the extending direction of the second casing 14B). The tubular portion 250 is provided in such a manner that the tubular portion 250 is accommodated in the second casing 14B. The acting member 212 is inserted into the tubular portion 250 in such a manner that the acting member 212 is disposed at the center of the tubular portion 250. The acting member 212 is formed as a shaft member having a circular cross section. The gas flow channel 222 is defined by an inner surface portion (inner wall portion) of the tubular portion 250 and an outer surface portion of the acting member 212 (the shaft member). The inert gas flows through the space between the tubular portion 250 and the acting member 212 from the proximal end side of the second casing 14B (the first casing 14A side) toward the distal end side thereof.

As shown in FIG. 5, the electric field generation section 230 has first electrode portions 231, second electrode portions 232, and dielectric members 240 which are partially interposed between the first electrode portions 231 and the second electrode portions 232. The electric field generation section 230 is incorporated into the handpiece 203. This electric field generation section 230 functions to generate an electric field within the gas flow channel 222 by using a potential difference between the first electrode portions 231 and the second electrode portions 232, thereby producing low-temperature plasma discharge. The electric field generation section 230 operates to produce low-temperature plasma discharge in the elongated handpiece 203 at a position near one end thereof in the longitudinal direction (a position near the distal end of the acting member 212). Specifically, the electric field generation section 230 produces low-temperature plasma discharge at a position near the distal end of the second casing 14B (the extension portion) (a position on the distal end side of the longitudinal center of the second casing 14B).

As shown in FIG. 6, the electric field generation section 230 includes a plurality of plate-shaped dielectric members 240 disposed in an annular pattern. A portion of each dielectric member 240 partially forms the inner wall of the gas flow channel 222. The second electrode portion 232 is embedded in each dielectric member 240, and the first electrode portion 231 is disposed in the dielectric member 240 to be located on the side toward the gas flow channel 222 with respect to the second electrode portion 232. A power supply unit similar to the power supply unit 9 shown in FIG. 1 is used so as to apply an AC voltage having a predetermined frequency between the first electrode portions 231 and the second electrode portions 232. This power supply unit 9 operates to maintain each first electrode portion 231 at the ground potential and oscillationally change the potential of each second electrode portion 232 within a range between a potential of +A (V) and a potential of -A (V). The potential of +A (V) is higher than the potential of the first electrode portions 231 (which is the ground potential and is the center of the range) by a predetermined amount. The potential of -A (V) is lower than the potential of the first electrode portions 231 by a predetermined amount. Notably, "A" is a positive value. When the AC voltage having a predetermined frequency is applied, creeping discharge occurs along the surfaces of the dielectric members 240 on the side toward the gas flow channel 222. Notably, in this example as well, for example, alumina is preferably used for the dielectric members 240.

In present structure as well, the second electrode portions 232 are electrodes whose potential can change to be higher than the potential of the first electrode portions 231, and each of the dielectric members 240 of the plurality of wall portions 250A, 250B, 250C, and 250D, has the first dielectric member portion 241 disposed on a surface of the second electrode portion 232 on the side toward the first electrode portion 231, and the second dielectric member portion 242 disposed on the surface of the second electrode portion 232 on the side opposite the surface on the side toward the first electrode portion 231. As shown in FIG. 6, the thickness T4 of the second dielectric member portion 242 is greater than the thickness T3 of the first dielectric member portion 241. The thickness T3 corresponds to the spacing between each first electrode portion 231 and the corresponding one of the second electrode portions 232, and the thickness T4 is equal to the spacing between the second electrode portion 232 in each dielectric member 240 and the surface 242A of the dielectric member 240 on the side opposite the gas flow channel 222.

As shown in FIG. 6, in the plasma irradiation device 220, the first electrode portions 231 are disposed around the gas flow channel 222 in an intermittent annular pattern. Also, the second electrode portions 232 are disposed, in an intermittent annular pattern, around the gas flow channel 222 and the first electrode portions 231 disposed in an annular pattern. Specifically, as shown in FIG. 6, the wall portions 250A, 250B, 250C, and 250D of the tubular portion 250 are configured such that the first electrode portion 231 and the second electrode portion 232 are disposed in each of the dielectric members 240. The wall portions 250A, 250B, 250C, and 250D have the same structure, and the tubular portion 250 is formed by connecting these wall portions 250A, 250B, 250C, and 250D to form an annular shape.

In the plasma irradiation device 220 configured as described above, the inner wall of the gas flow channel 222 is formed by the dielectric members 240 at the respective wall portions 250A, 250B, 250C, and 250D of the tubular portion 250, and, at each wall portion 250A, 250B, 250C, or 250D, creeping discharge occurs in the vicinity of the inner wall surface (the boundary surface between the wall portion and the space of the gas flow channel 222). Specifically, as shown in FIG. 6, the second electrode portions 232, which are wider than the first electrode portions 231, are disposed to face the corresponding first electrode portions 231, with the dielectric members 240 intervening therebetween. Each second electrode portion 232 extends such that portions of the second electrode portion 232 on the laterally opposite sides extend beyond opposite lateral ends of the corresponding first electrode portion 231. The first electrode portions 231 are disposed near the front surfaces (surfaces on the side toward the gas flow channel 222) of the dielectric members 240. Notably, here, of directions perpendicular to the axial direction of the acting member 212, a direction in which the first electrode portion 231 in each dielectric member 240 extends will be referred to as the width direction of the first electrode portion 231, and a direction in which the second electrode portion 232 in the dielectric member 240 extends will be referred to as the width direction of the second electrode portion 232. In this structure, creeping surfaces of the dielectric member 240 are present on the opposite sides of the first electrode portion 231 in the width direction. These creeping surfaces face portions of the second electrode portion 232 near its opposite ends in the width direction. In such a structure, changes in electric fields stemming from the potential difference between the first electrode portions 231 and the second electrode portions 232 easily occur in the vicinity of the creeping surfaces of the dielectric members 240 (the boundary surfaces between the dielectric members 240 and the space of the gas flow channel 222). Therefore, when the AC voltage is applied between the first electrode portions 231 and the second electrode portions 232, strong electric fields are induced along the creeping surfaces of the dielectric members 240 (creeping surfaces of the gas flow channel 22 through which the inert gas flows), whereby creeping discharge occurs. In the gas flow channel 222, the inert gas flows toward an end portion of the gas flow channel 222 (specifically, the opening portion 52 forming an end portion of the tubular portion 50 shown in FIG. 2). The low-temperature plasma produced as a result of the creeping discharge is discharged from the end portion of the gas flow channel 222 (specifically, the opening portion 252 which is a distal end portion of the tubular portion 250) toward the distal end side of the acting member 212. Since the handpiece 203 has the structure as described above, the operator can irradiate a bleeding portion with the low-temperature plasma by operating the handpiece 203 such that its distal end portion (a distal end portion of the acting member 212) is directed toward the bleeding portion and by activating the plasma irradiation device 220. As a result, blood coagulation occurs, whereby a stanching treatment can be performed.

Next, a method, which is not claimed, of manufacturing the plasma irradiation device 220 will be described. Here, a method of manufacturing the wall portions (wall portions 250A, 250B, 250C, and 250D) constituting the tubular portion 250 shown in FIG. 6 will be mainly described.

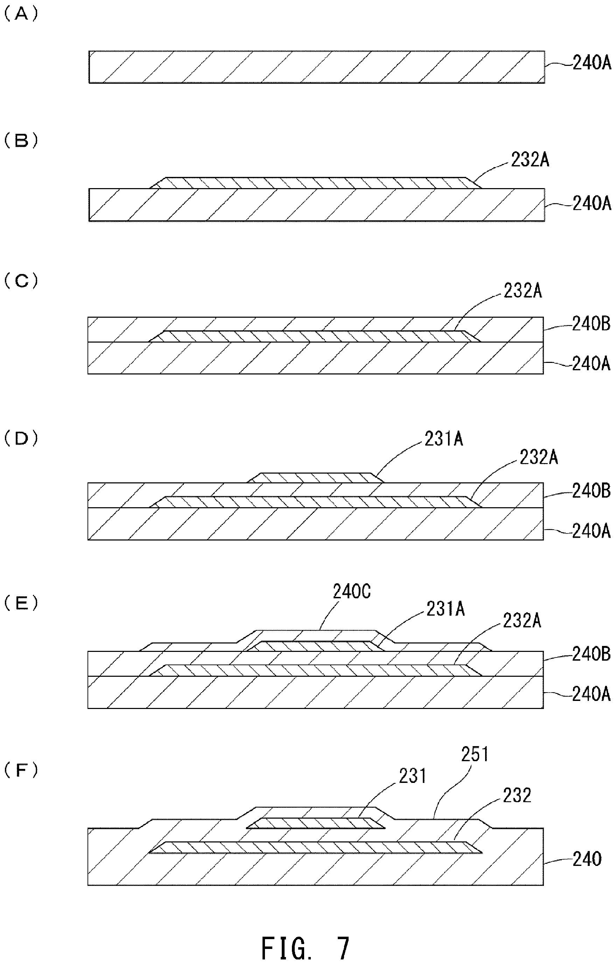

For manufacture of the wall portions constituting the tubular portion 250, a first ceramic green sheet formation step is performed first. As shown in FIG. 7(A), in the first ceramic green sheet formation step, a first ceramic green sheet 240A having a predetermined thickness is formed by using a ceramic material containing alumina powder as a main component. After the first ceramic green sheet formation step, a first unfired conductive layer formation step is performed. In the first unfired conductive layer formation step, as shown in FIG. 7(B), a tungsten paste 232A containing tungsten as a main component is applied (printed) on one side of the first ceramic green sheet 240A by using a paste printing device. After the first unfired conductive layer formation step, a second ceramic green sheet formation step is performed. In the second ceramic green sheet formation step, a second ceramic green sheet 240B having a predetermined thickness is formed by using a ceramic material containing alumina powder as a main component, and, as shown in FIG. 7(C), this second ceramic green sheet 240B is placed on a laminate obtained in the first unfired conductive layer formation step and is compression-bonded thereto. After the second ceramic green sheet formation step, a second unfired conductive layer formation step is performed. In the second unfired conductive layer formation step, as shown in FIG. 7(D), a tungsten paste 231A containing tungsten as a main component is applied (printed) on one side of the second ceramic green sheet 240B by using a paste printing device. After the second unfired conductive layer formation step, a ceramic protection layer formation step is performed. In the ceramic protection layer formation step, as shown in FIG. 7(E), an alumina paste 240C is applied (printed), by using a paste printing device, on one side of the laminate obtained in the second unfired conductive layer formation step so as to cover the tungsten paste 231A. After the ceramic protection layer formation step, a firing step is performed. In the firing step, firing is performed for the laminate obtained in the ceramic protection layer formation step. As a result of this firing step, as shown in FIG. 7(F), alumina in the ceramic green sheets, tungsten in the tungsten paste, and alumina in the alumina paste sinter, whereby a plate-shaped member 251 (a plate-shaped member in which a first electrode portion 231 and a second electrode portion 232 are embedded in a dielectric member 240) is produced. Notably, the first electrode portion 231 and the second electrode portion 232 of the plate-shaped member 251 can be electrically connected to an external circuit in the same manner as for the second electrode portion 32 of the first embodiment.

A plurality of plate-shaped members 251 (FIG. 7(F)) which are to become the wall portions of the tubular portion 250 are formed through the above-described steps and are disposed annularly, whereby at least a portion of the tubular portion 250 (an annular portion in which the first and second electrode portions 231 and 232 are embedded as shown in FIG. 6) can be formed. Each of the wall portions 250A, 250B, 250C, and 250D shown in FIG. 6 is formed by the plate-shaped member 251 as shown in FIG. 7(F). The tubular portion 250 may be formed into a square tubular shape through use of only the plurality of plate-shaped members 251. Alternatively, the tubular portion 250 may be formed by connecting another annular member (for example, a square tubular member made of an insulating material which is the same as or different from the material of the dielectric members 240) to the plurality of plate-shaped members 251 forming a squire tubular shape. After formation of the tubular portion 250, the acting member 212 is disposed inside the tubular portion 250, and the tubular portion 250 and the acting member 212 are held such that they have a predetermined positional relation therebetween, whereby the plasma irradiation device 220 is obtained.

As described above, in the plasma irradiation device 220 of the present structure, the portions of the dielectric members 240 located between the first electrode portions 231 and the second electrode portions 232 are in contact with the surfaces of the first electrode portions 231 and the surfaces of the second electrode portions 232. At least portions of the dielectric members 240 constitute the inner wall of the gas flow channel 222, and low-temperature plasma discharge is produced along the inner wall. By virtue of this structure, it is possible to produce low-temperature plasma discharge along the inner wall surface of the gas flow channel 222 (the surfaces of the dielectric members 240 on the side toward the gas flow channel 222) and to efficiently supply low-temperature plasma produced as a result of the discharge toward the distal end portion side of the acting member 212 by utilizing the flow of the gas within the gas flow channel 222. Also, since the low-temperature plasma discharge can be produced in a relatively narrow region along the surfaces of the dielectric members 240, size reduction is easily realized.

Also, each dielectric member 240 has the first dielectric member portion 241 disposed on the surface of the corresponding second electrode portion 232 located on the side toward the corresponding first electrode portion 241, and the second dielectric member portion 242 disposed on the surface of the corresponding second electrode portion 232 located on the side opposite the surface on the side toward the first electrode portion 231. The second electrode portion 232 serves as an electrode whose potential oscillates such that the potential of the first electrode portion 231 (the ground potential) becomes the center of the potential oscillation. The thickness T4 of the second dielectric member portion 242 is greater than the thickness T3 of the first dielectric member portion 241. In the case where the thickness T4 of the second dielectric member portion 242 is greater than the thickness T3 of the first dielectric member portion 241 as described above, even when the potential of the second electrode portions 232 becomes high due to the oscillation of the potential, the influence of the high potential becomes unlikely to reach a region on the outer side of the second dielectric member portion 242 (a region on the side opposite the gas flow channel 222). As a result, a problem caused by the potential of the second electrode portions 232 becomes less likely to occur in the region on the outer side of the second dielectric member portion 242 (for example, on the outer side of the second casing 14B). In contrast, the influence of the potential of the second electrode portion 232 becomes more likely to reach the gas flow channel 222, so that the field intensity can be increased more easily within the gas flow channel 222.

Also, the first electrode portions 231 are disposed around the acting member 212 in an intermittent annular pattern, and the second electrode portions 232 are disposed, in an intermittent annular pattern, around the first electrode portions 231 disposed in an annular pattern. Since the first electrode portions 231 and the second electrode portions 232 are annularly disposed around the acting member 212 as described above, a wider region for generation of low-temperature plasma discharge can be secured around the acting member 212.

### <Third embodiment, covered by the claimed invention>

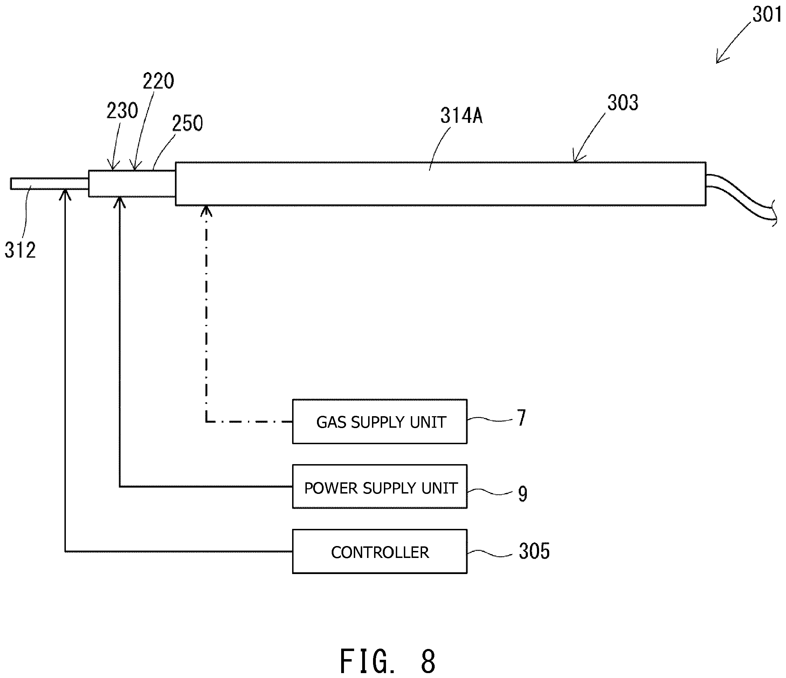

A plasma irradiation device 220 of a third embodiment shown in FIG. 8 has the same structure as the plasma irradiation device 220 of the second embodiment shown in FIG. 5, etc., and is applied to a surgical operation device 301 which functions as an electric knife. In the example of FIG. 8, an acting member 312 is used instead of the acting member 212 (FIG. 5, etc.). The arrangement and shape of the acting member 312 are identical with those of the acting member 212 of the plasma irradiation device 220. The acting member 312 differs from the acting member 212 in the point that high frequency current is supplied instead of ultrasonic vibration.

In the surgical operation device 301 to which the plasma irradiation device 220 of the third embodiment is applied, a handpiece 403 is configured such that a grip portion 314A formed as a cylindrical case is provided and the plasma irradiation device 220 (see FIG. 5, etc.) is disposed to extend from the grip portion 314A. The acting member 312 is inserted into the tubular portion 250 of the plasma irradiation device 220. A gas flow channel is formed between the inner wall of the tubular portion 250 and the acting member 312. Low-temperature plasma discharge is generated in this gas flow channel, and plasma is supplied to the distal end portion side of the acting member 312.

In the third embodiment, a controller 305 corresponding to the drive section is configured as a high-frequency-current supply section for supplying high frequency current, and the acting member 312 functions as an electrode portion through which the high frequency current supplied from the controller 305 (high-frequency-current supply section) flows. Namely, the acting member 312 can function as a known electric knife. The acting member 312 is configured to provide an incising action, an ablating action, or a thermocoagulation stanching action for biological tissue by utilizing the high frequency current flowing through the acting member 312 (the electrode portion). This plasma irradiation device 220 allows an operator to perform, through use of the common handpiece 303, incision, ablation, or hemostasis (through thermocoagulation) of biological tissue by the high frequency current flowing through the acting member 312 (the electrode portion), as well as minimally invasive hemostasis by irradiation with low-temperature plasma. Notably, in the example of FIG. 8, the controller 305 corresponding to an example of the drive section is provided externally of the grip portion 314A formed as a cylindrical case and is configured as a high-frequency-current supply section for supplying high frequency current. However, the high-frequency-current supply section (for example, a high-frequency-current generation circuit) for supplying high frequency current may be disposed inside the grip portion 314A and function as the drive section.

### <Fourth embodiment,covered by the claimed invention>

Next, a plasma irradiation device 420 of a fourth embodiment will be described.

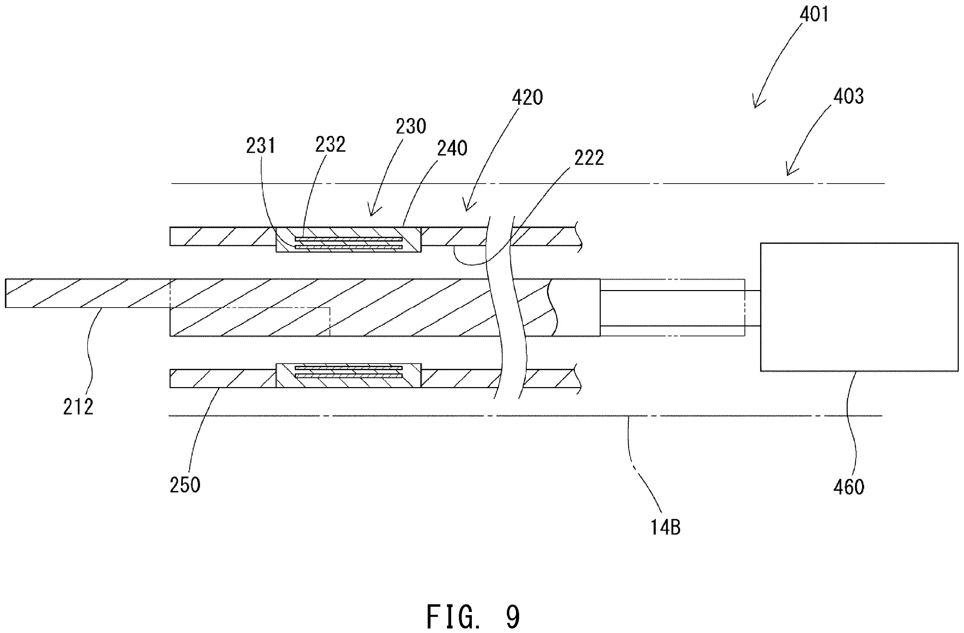

The plasma irradiation device 420 of the fourth embodiment shown in FIG. 9 is identical with the plasma irradiation device 220 of the second embodiment except for addition of a displacement device 460. A surgical operation device 401 shown in FIG. 9 is identical with the surgical operation device 1 shown in FIG. 1, etc. except for the point that the surgical operation device 401 includes a plasma irradiation device 420 instead of the plasma irradiation device 20. The handpiece 403 shown in FIG. 9 is identical with the handpiece 3 shown in FIG. 1, etc. except for the point that the handpiece 403 includes the plasma irradiation device 420 instead of the plasma irradiation device 20.

The plasma irradiation device 420 shown in FIG. 9 includes a displacement device 460 which moves the acting member 212 to a projecting position at which the acting member 212 projects from a main body portion of the handpiece 403 (specifically, a portion remaining after exclusion of the acting member 212 from the handpiece 403) and a retracted position at which the amount of projection of the acting member 212 is smaller than that at the projecting position. The displacement device 460 is a known linear actuator and can move the acting member 212 in the axial direction of the acting member 212. The displacement device 460 moves the acting member 212 to the projecting position shown in FIG. 9 when the displacement device 460 receives a first signal from, for example, an unillustrated control circuit. When the acting member 212 is located at the projecting position, the acting member 212 projects from the second casing 14B by a predetermined amount. When the displacement device 460 receives a second signal from the unillustrated control circuit, the displacement device 460 moves the acting member 212 to the retracted potion (position indicated by an alternate long and two short dashes line in FIG. 9) at which the amount of projection of the acting member 212 is smaller than that at the projecting position. By virtue of the above-described configuration, when necessary, the acting member 212 can be retracted, and the stanching treatment through application of low-temperature plasma can be performed in a state in which the acting member 31 is located at the retracted position.

### <Fifth embodiment, not covered by the claimed invention>

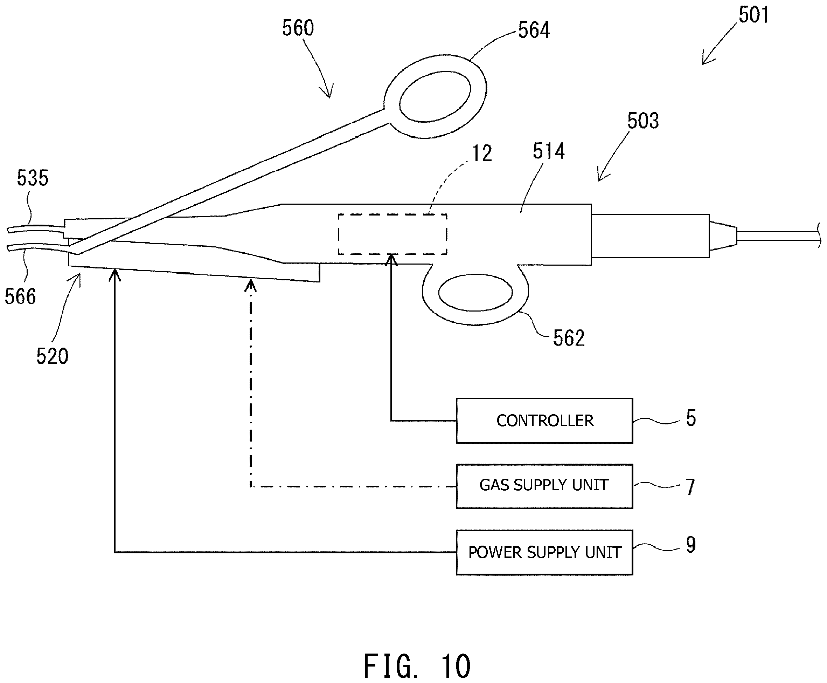

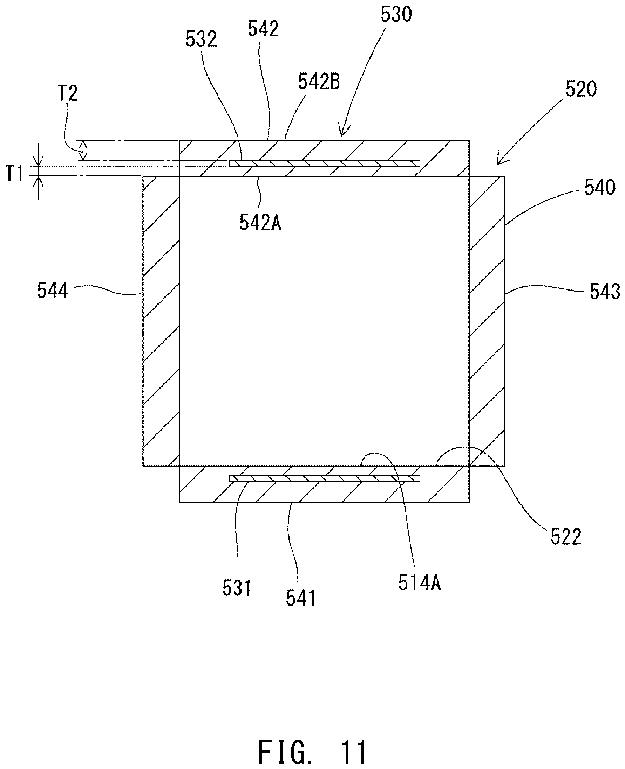

Next, a plasma irradiation device 520 of a fifth embodiment and a surgical operation device 501 including the plasma irradiation device 520 will be described with reference to mainly FIGS. 10 and 11. Notably, in the structure of FIGS. 10 and 11, portions having the same structures as those of the plasma irradiation device 20 of the first embodiment are denoted by the same symbols as those of the corresponding portions of the plasma irradiation device 20, and their detailed descriptions will be omitted.

As shown in FIG. 10, the surgical operation device 501 which includes the plasma irradiation device 520 of the fifth embodiment includes a handpiece 503 into which an acting member 535 acting on biological tissue is incorporated; the controller 5 for controlling the ultrasonic vibration section 12 (the drive section); the gas supply unit 7 which supplies a gas to the gas flow channel 22; and the power supply unit 9 which can apply voltage to the plasma irradiation device 520. The controller 5, the gas supply unit 7, the power supply unit 9, and the ultrasonic vibration section 12 provided in the surgical operation device 501 have configurations identical with those of the controller 5, the gas supply unit 7, the power supply unit 9, and the ultrasonic vibration section 12 of the surgical operation device 1 shown in FIG. 1 and function in the same manners as the controller 5, the gas supply unit 7, the power supply unit 9, and the ultrasonic vibration section 12 of the surgical operation device 1.

The surgical operation device 501 shown in FIG. 10 is an apparatus which is preferably used in, for example, surgical operation. The surgical operation device 501 has at least a function of incising or ablating biological tissue or performing hemostasis through thermocoagulation by utilizing ultrasonic vibration. The surgical operation device 501 is configured to allow an operator to perform incision, ablation, or hemostasis for the biological tissue of an operation target. The surgical operation device 501 differs from the surgical operation devices described in the first embodiment, etc., in the point that the acting member 535 acting on the biological tissue is disposed outside the gas flow channel 522. The ultrasonic vibration section 12 is configured to vibrate the acting member 535 disposed outside the flow channel.

The handpiece 503 mainly includes the plasma irradiation device 520, the ultrasonic vibration section 12, a movable member displacement mechanism 560, a casing 514, etc. The ultrasonic vibration section 12, etc. are accommodated in the casing 514. The acting member 535 is a member whose distal end portion acts on the biological tissue as a stationary blade. The acting member 535 corresponds to an example of the vibration member to which the ultrasonic vibration generated by the ultrasonic vibration section 12 is transmitted. The movable member displacement mechanism 560 is a mechanism which displaces a movable member 566 functioning as a movable blade and employs a known movable mechanism. This movable member displacement mechanism 560 includes a stationary grip portion 562 fixed to the casing 514; and a movable grip portion 564 attached to be movable relative to the stationary grip portion 562. The rod-shaped movable member 566 is pivotable about a pivot axis near a distal end portion of the casing 514. When an operation of moving the movable grip portion 564 toward the stationary grip portion 562 is performed, the movable member 566 pivots in such a manner that a distal end portion of the movable member 566 moves toward a distal end portion of the acting member 535. In contrast, when an operation of separating the movable grip portion 564 from the stationary grip portion 562 is performed, the movable member 566 pivots in such a manner that the distal end portion of the movable member 566 moves away from the distal end portion of the acting member 535.

As shown in FIG. 10, the plasma irradiation device 520 is incorporated into the handpiece 3 and is configured as an apparatus which produces dielectric barrier discharge. As shown in FIG. 11, the plasma irradiation device 520 includes a gas flow channel 522 and an electric field generation section 530. The inert gas supplied from the outside of the handpiece 503 (specifically, the gas supply unit 7 shown in FIG. 10) is supplied to a distal end portion of the acting member 535 (FIG. 10) through the gas flow channel 522. The electric field generation section 530 is disposed in the gas flow channel 522 and generates an electric field in the space within the gas flow channel 522 by using a potential difference between the first electrode portion 531 and the second electrode portion 532, thereby producing low-temperature plasma discharge.

The electric field generation section 530 operates to produce low-temperature plasma in the elongated handpiece 503 (FIG. 10) at a position near one end thereof in the longitudinal direction (a position near the distal end of the acting member 535 shown in FIG. 10). As shown in FIG. 11, the electric field generation section 530 includes the first electrode portion 531, the second electrode portion 532 facing the first electrode portion 531, and a dielectric member 540, a portion of which is located between the first electrode portion 531 and the second electrode portion 532. The first electrode portion 531 is an electrode formed as a ground electrode and is maintained at the ground potential during use (at the time of generation of low-temperature plasma discharge). The second electrode portion 532 is an electrode to which an AC voltage having a predetermined frequency is applied by the power supply unit 9 and whose potential oscillates such that the potential of the first electrode portion 531 (the ground potential) becomes the center of the potential oscillation during use (at the time of generation of low-temperature plasma discharge).

The dielectric member 540 constitutes a plurality of wall portions 541, 542, 543, and 544, and these wall portions 541, 542, 543, and 544 define a square gas flow channel 522. The inner wall surface of the gas flow channel 522 is formed by the wall surfaces of the wall portions 541, 542, 543, and 544. The wall portion 542 is a wall portion (a wall portion formed by the dielectric member) in which the second electrode portion 532 is embedded. The wall portion 542 includes a first dielectric member portion 542A disposed on a surface of the second electrode portion 532 located on the side toward the first electrode portion 531, and a second dielectric member portion 542B disposed on a surface of the second electrode portion 532 located on the side opposite the surface on the side toward the first electrode portion 531. The thickness T2 of the second dielectric member portion 542B is greater than the thickness T1 of the first dielectric member portion 542A. The dielectric member 540 is configured such that each of portions located between the first electrode portion 531 and the second electrode portion 532 is in contact with only one of the surface of the first electrode portion 531 and the surface of the second electrode portion 532. For example, a portion 541A located between the first electrode portion 531 and the second electrode portion 532 in the wall portion 541 is in contact with only the surface of the first electrode portion 531, and a portion (a first dielectric member portion 542A) located between the first electrode portion 531 and the second electrode portion 532 in the wall portion 542 is in contact with only the surface of the second electrode portion 532. The space within the gas flow channel 522 is present between the first electrode portion 531 and the second electrode portion 532, and the electric field generation section 530 produces low-temperature plasma discharge (space discharge) in the space.

In the plasma irradiation device 520 configured as described above, an AC voltage having a predetermined frequency is applied between the first electrode portion 531 and the second electrode portion 532 by the power supply unit 9 in a state in which the inert gas supplied from the gas supply unit 7 flows through the space within the gas flow channel 522. As a result, in a state in which the dielectric member 540 forms barriers on the first electrode portion 531 and the second electrode portion 532, changes in electric fields occur between these electrodes, whereby dielectric barrier discharge occurs in the space within the gas flow channel 522. The low-temperature plasma produced as a result of the dielectric barrier discharge is discharged from one end portion of the gas flow channel 522 toward the distal end side of the acting member 535.

Notably, in the example of FIG. 11, the gas flow channel 522 has a quadrangular cross section when cut in a planar direction perpendicular to the extension direction of the gas flow channel 522. However, the cross section may have a polygonal shape other than the quadrangular shape, such as a circular shape, an elliptical shape, or the like. Also, in the example of FIG. 11, the dielectric member 540 has the portion 541A in contact with only the first electrode portion 531 and the portion (the first dielectric member portion 542A) in contact with only the second electrode portion 532. However, one of the portion 541A and the first dielectric member portion 542A may be omitted. Namely, it is sufficient that the gas flow channel side of at least one of the first and second electrode portions (electrodes) 531 and 532 is covered with the dielectric member, and the dielectric member is not required to cover both the electrodes. Also, in the example of FIG. 11, one pair of the first electrode portion 531 and the second electrode portion 532 is provided. However, another pair of the first electrode portion 531 and the second electrode portion 532 may be provided in, for example, in the wall portions 543 and 544.

### <Sixth embodiment, covered by the claimed invention>

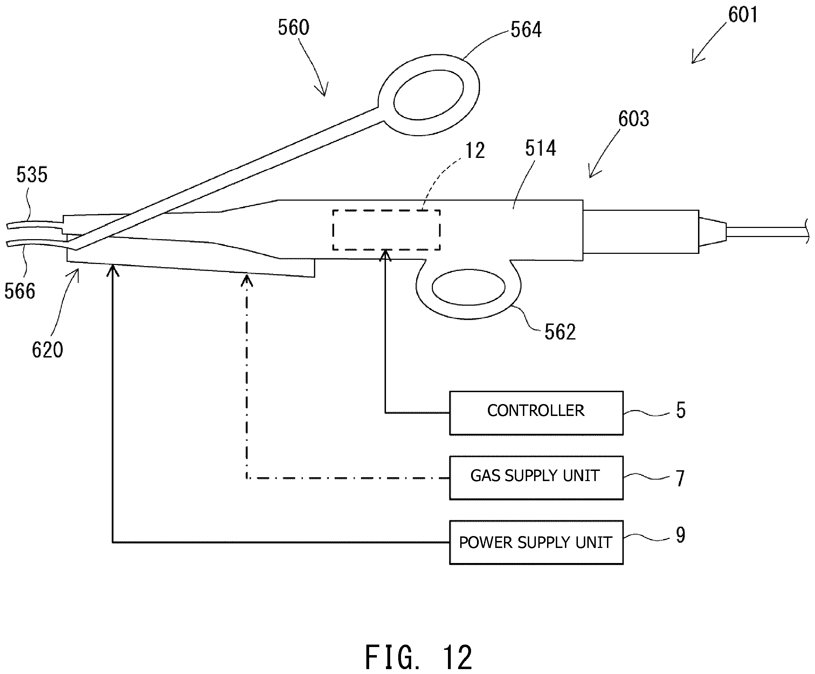

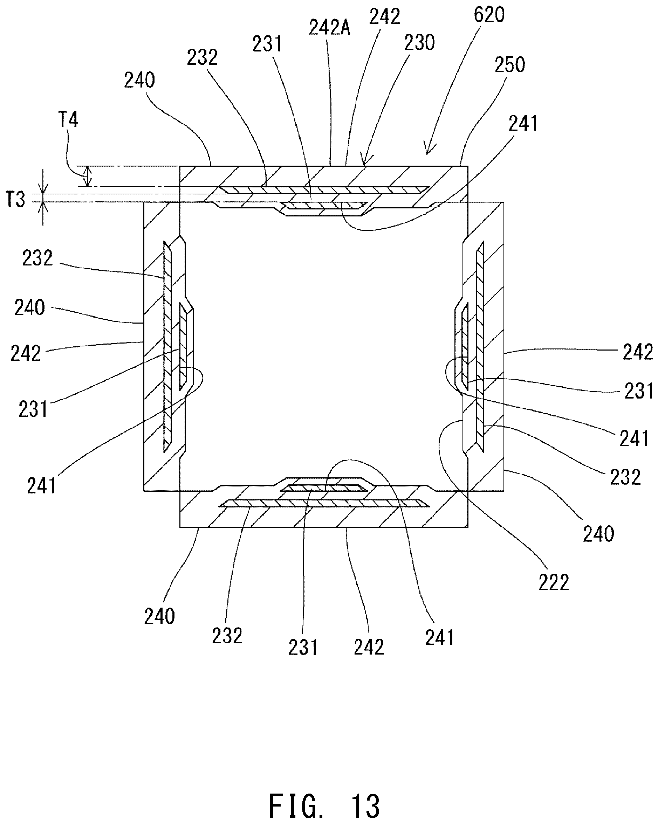

Next, a sixth embodiment will be described with reference to FIGS. 12 and 13. A surgical operation device 601 of the sixth embodiment shown in FIG. 12 differs from the surgical operation device 501 of the fifth embodiment only in the point that, in the surgical operation device 501 shown in FIG. 10, a plasma irradiation device 620 shown in FIG. 13 is used in place of the plasma irradiation device 520. Therefore, in FIG. 12, portions having the same structures as those of the surgical operation device 501 of the fifth embodiment shown in FIG. 10 are denoted by the same symbols as those of the corresponding portions of the surgical operation device 501, and their detailed descriptions will be omitted. Also, the plasma irradiation device 620 shown in FIG. 13 differs from the plasma irradiation device 220 of the second embodiment only in the point that the acting member 212 is omitted from the plasma irradiation device 220 of the second embodiment shown in FIG. 5, FIG. 6, etc. Therefore, in FIG. 13, portions having the same structures as those of the plasma irradiation device 220 of the second embodiment shown in FIG. 5, FIG. 6, etc. are denoted by the same symbols as those of the corresponding portions of the plasma irradiation device 220, and their detailed descriptions will be omitted.

The plasma irradiation device 620 shown in FIG. 13 has the same structure as the plasma irradiation device 220, and portions of the dielectric members 240 located between the first electrode portions 231 and the second electrode portions 232 are in contact with both the surfaces of the first electrode portions 231 and the surfaces of the second electrode portions 232. Further, the inner wall portions of the gas flow channel 222 are formed by the dielectric members 240, and the electric field generation section 230 produces low-temperature plasma discharge (creeping discharge) along the inner wall portions. Also, each dielectric member 240 has a first dielectric member portion 241 disposed on the surface of the corresponding second electrode portion 232 on the first electrode portion 231 side, and a second dielectric member portion 242 disposed on the surface of the corresponding second electrode portion 232 on the side opposite the surface on the first electrode portion 231 side. The thickness T4 of the second dielectric member portion 242 is larger than the thickness T3 of the first dielectric member portion 241. Also, the first electrode portions 231 are disposed in an intermittent annular pattern, and the second electrode portions 232 are disposed in an intermittent annular pattern around the first electrode portions 231 disposed in the annular pattern.

### <Other examples>

In the first embodiment, the second electrode portions 32 are disposed in an intermittent annular pattern. However, the second electrode portions 32 may be disposed in a continuous annular pattern. For example, a second electrode portion having a square tubular shape may be embedded in a second-electrode-portion-side dielectric member having a square tubular shape. Alternatively, a second electrode portion having a circular tubular shape may be embedded in a second-electrode-portion-side dielectric member having a circular tubular shape.

In the second and sixth embodiments, the first electrode portions 231 are disposed in an intermittent annular pattern. However, the first electrode portions 231 may be disposed in a continuous annular pattern, so long as creeping discharge is produced. Also, the second electrode portions 232 are disposed in an intermittent annular pattern. However, the second electrode portions 232 may be disposed in a continuous annular pattern, so long as creeping discharge is produced.

In the first, second, fifth, and sixth embodiments, the drive section is disposed inside the casing of the handpiece. However, the drive section may be disposed outside the casing of the handpiece as in the third embodiment. In such a case as well, the drive section is considered to be part of the handpiece.

In the third embodiment or the fourth embodiment, the discharge scheme used in the first embodiment may be used.

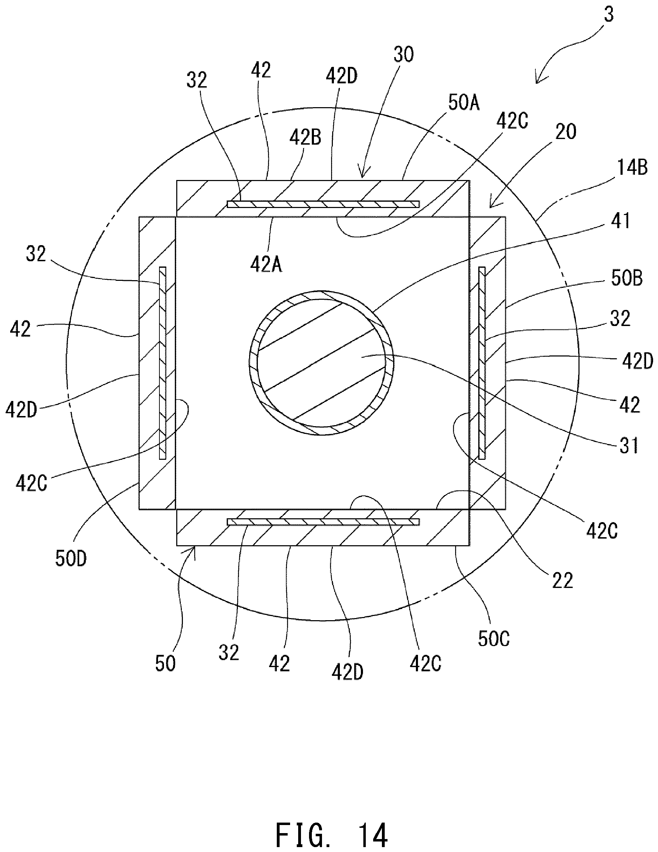

A portion of the plasma irradiation device 20 of the first embodiment may be modified as shown in FIG. 14. The example of FIG. 14 differs from the plasma irradiation device 20 of the first embodiment in the point that the acting member 31 is formed as a shaft member having a circular cross section, and a first-electrode-portion-side dielectric member 41 having a circular tubular shape is disposed around the acting member 31.

In the example structures shown by FIG. 3, FIG. 6, FIG. 13, etc., the dielectric members and the second electrode portions are disposed in a square pattern. However, in any example, the dielectric members and/or the second electrode portions may be disposed in any polygonal pattern other than the square pattern. Also, a circular-tubular dielectric member may be employed. In this case, a second electrode portion(s) may be disposed in a continuous or intermittent annular pattern. In the case where the dielectric member has a circular tubular shape, an electric field generation section having a circular tubular shape may be formed by rolling up, into a circular tubular shape, a laminate of ceramic sheets with tungsten paste therebetween by using a jig or the like before firing, and firing the rolled laminate while supporting it by using a jig or the like so as to prevent deformation of the rolled laminate.

In the claims and specification, the expression "acting on biological tissue" means that the acting member influences the biological tissue, thereby performing at least one of incision, ablation, and hemostasis. The acting member used in the above-described embodiments is merely an example, and acting members having various structures other than the acting member used in the above-described embodiments may be employed, so long as the employed acting member influences the biological tissue, thereby performing at least one of incision, ablation, and hemostasis.

### DESCRIPTION OF REFERENCE NUMERALS

1, 201, 301, 401, 501, 601 ··· surgical operation device
3, 203, 303, 403, 503, 603 ··· handpiece
12 ··· ultrasonic vibration section (drive section)
20, 220, 420, 520, 620 ··· plasma irradiation device
22, 222, 522 ··· gas flow channel
30, 230, 530 ··· electric field generation section
31 ··· acting member (first electrode portion)
32 ··· second electrode portion
40, 240, 540 ··· dielectric member
42C ··· first dielectric member portion
42D ··· second dielectric member portion
50, 250 ··· tubular portion
212 ··· acting member
231 ··· first electrode portion
232 ··· second electrode portion
241 ··· first dielectric member portion
242 ··· second dielectric member portion
305 ··· controller (drive section, high-frequency-current supply section)
312 ··· acting member
460 ··· displacement device
531 ··· first electrode portion
532 ··· second electrode portion
535 ··· acting member
542A ··· first dielectric member portion
542B ··· second dielectric member portion

## Claims

1. A plasma irradiation device (20, 220, 420, 520, 620) provided in a handpiece (3, 203, 303, 403, 503, 603) including an acting member (31, 212, 312, 535) which acts on biological tissue, comprising:
a gas flow channel (22, 222, 522) for supplying to a distal end portion of the acting member (31, 212, 312, 535) a gas supplied externally of the handpiece (3, 203, 303, 403, 503, 603); and
an electric field generation section (30, 230, 530) disposed in the gas flow channel (22, 222, 522), the electric field generation section (30, 230, 530) including a first electrode portion (31, 231, 531), a second electrode portion (32, 232, 532) facing the first electrode portion (31, 231, 531), and a dielectric member (40, 240, 540) having at least a portion which is located between the first electrode portion (31, 231, 531) and the second electrode portion (32, 232, 532) and is disposed on at least one of a surface of the first electrode portion (31, 231, 531) and a surface of the second electrode portion (32, 232, 532), the electric field generation section (30, 230, 530) is configured to generate an electric field in a space within the gas flow channel (22, 222, 522) by using a potential difference between the first electrode portion (31, 231, 531) and the second electrode portion (32, 232, 532), thereby producing the low-temperature plasma discharge,
**characterized in that**
the dielectric member (240) is configured such that the portion located between the first electrode portion (231) and the second electrode portion (232) is in contact with both the surface of the first electrode portion (231) and the surface of the second electrode portion (232); at least a portion of the dielectric member (240) forms an inner wall portion of the gas flow channel (222); and the low-temperature plasma discharge is produced along the inner wall portion.

2. A plasma irradiation device (220, 420, 620) according to claim 1, wherein the dielectric member (240) has a first dielectric member portion (241) disposed on a surface of the second electrode portion (232) on a side toward the first electrode portion (231) and a second dielectric member portion (242) disposed on a surface of the second electrode portion (232) on a side opposite the surface on the side toward the first electrode portion (231);
the second electrode portion (232) is an electrode whose potential oscillates such that a potential of the first electrode portion (231) becomes the center of the potential oscillation; and
the second dielectric member portion (242) has a thickness (T4) greater than a thickness (T3) of the first dielectric member portion (241).

3. A plasma irradiation device (220, 420, 620) according to claim 1 or 2, wherein
the first electrode portion (231) is disposed in a continuous or intermittent annular pattern; and
the second electrode portion (232) is disposed in a continuous or intermittent annular pattern around the first electrode portion (231) disposed in the annular pattern.

4. A plasma irradiation device (20, 220, 420) according to any one of claims 1 to 3, further comprising a tubular portion (50, 250) which includes the acting member (31, 212, 312) provided therein and extending in a predetermined direction,
wherein the acting member (31, 212, 312) has a rod-like shape, and one end portion of the acting member (31, 212, 312) serves as an acting portion acting on biological tissue.

5. A plasma irradiation device (20, 220, 420) according to claim 4, wherein the electric field generation section (30, 230) is provided in the tubular portion (50, 250) to be located at a position corresponding to the one end portion of the acting member (31, 212, 312).

6. A handpiece (3, 203, 503, 603) comprising:
a plasma irradiation device (20, 220, 520, 620) according to any one of claims 1 to 5; and
a drive section (12) for driving the acting member (31, 212, 535), wherein
the drive section (12) is an ultrasonic vibration section for generating ultrasonic vibration; and
the acting member (31, 212, 535) vibrates as a result of transmission of the ultrasonic vibration generated by the ultrasonic vibration section to the acting member, thereby performing an incising action, an ablating action, or a stanching action for the biological tissue.

7. A handpiece (303) comprising:
a plasma irradiation device (220) according to any one of claims 1 to 5; and
a drive section (305) for driving the acting member (312), wherein
the drive section (305) is a high-frequency current supply section for supplying high-frequency current; and
as a result of the high-frequency current supplied from the high-frequency current supply section flowing through the acting member (312), the acting member (312) performs an incising action, an ablating action, or a stanching action for the biological tissue.

8. A handpiece (403) comprising:
a plasma irradiation device (420) according to claim 4 or 5; and
a displacement device (460) for moving the acting member (212) between a projecting position at which the acting member (212) projects from the tubular portion (250) and a retracted position at which the amount of projection of the acting member (212) is smaller than that at the projecting position.

9. A surgical operation device (1, 201, 301, 401, 501, 601) comprising a handpiece (3, 203, 303, 403, 503, 603) according to any one of claims 6 to 8.

## Patentansprüche

1. Plasmastrahlungsvorrichtung (20, 220, 420, 520, 620), die in einem Handstück (3, 203, 303, 403, 503, 603) vorgesehen ist, das ein Einwirkungselement (31, 212, 312, 535) aufweist, das auf biologisches Gewebe einwirkt, aufweisend:
einen Gasströmungskanal (22, 222, 522), um einem distalen Endabschnitt des Einwirkungselements (31, 212, 312, 535) ein Gas zuzuführen, das außerhalb des Handstücks (3, 203, 303, 403, 503, 603) zugeführt wird; und
einen Abschnitt zur Erzeugung eines elektrischen Felds (30, 230, 530), der in dem Gasströmungskanal (22, 222, 522) angeordnet ist, wobei der Abschnitt zur Erzeugung eines elektrischen Felds (30, 230, 530) einen ersten Elektrodenabschnitt (31, 231, 531), einen zweiten Elektrodenabschnitt (32, 232, 532), der dem ersten Elektrodenabschnitt (31, 231, 531) zugewandt ist, und ein dielektrisches Element (40, 240, 540), das mindestens einen Abschnitt aufweist, der sich zwischen dem ersten Elektrodenabschnitt (31, 231, 531) und dem zweiten Elektrodenabschnitt (32, 232, 532) angeordnet ist und auf einer Oberfläche des ersten Elektrodenabschnitts (31, 231, 531) und/oder einer Oberfläche des zweiten Elektrodenabschnitts (32, 232, 532) angeordnet ist, umfasst, wobei der Abschnitt zur Erzeugung eines elektrischen Felds (30, 230, 530) derart konfiguriert ist, dass er ein elektrisches Feld in einem Raum innerhalb des Gasströmungskanals (22, 222, 522) unter Verwendung einer Potentialdifferenz zwischen dem ersten Elektrodenabschnitt (31, 231, 531) und dem zweiten Elektrodenabschnitt (32, 232, 532) erzeugt, wodurch die Niedertemperatur-Plasmaentladung erzeugt wird, **dadurch gekennzeichnet, dass**
das dielektrische Element (240) derart konfiguriert ist, dass der zwischen dem ersten Elektrodenabschnitt (231) und dem zweiten Elektrodenabschnitt (232) angeordnete Abschnitt sowohl mit der Oberfläche des ersten Elektrodenabschnitts (231) als auch mit der Oberfläche des zweiten Elektrodenabschnitts (232) in Kontakt steht; mindestens ein Abschnitt des dielektrischen Elements (240) einen Innenwandabschnitt des Gasströmungskanals (222) bildet; und die Niedertemperatur-Plasmaentladung entlang des Innenwandabschnitts erzeugt wird.

2. Plasmastrahlungsvorrichtung (220, 420, 620) nach Anspruch 1, wobei das dielektrische Element (240) einen ersten dielektrischen Elementabschnitt (241), der auf einer Oberfläche des zweiten Elektrodenabschnitts (232) auf einer Seite zum ersten Elektrodenabschnitt (231) angeordnet ist, und einen zweiten dielektrischen Elementabschnitt (242), der auf einer Oberfläche des zweiten Elektrodenabschnitts (232) auf einer Seite gegenüber der Oberfläche auf der Seite zum ersten Elektrodenabschnitt (231) angeordnet ist, aufweist;
der zweite Elektrodenabschnitt (232) eine Elektrode ist, deren Potential derart oszilliert, dass ein Potential des ersten Elektrodenabschnitts (231) die Mitte der Potentialoszillation ist; und
der zweite dielektrische Elementabschnitt (242) eine Dicke (T4) aufweist, die größer ist als eine Dicke (T3) des ersten dielektrischen Elementabschnitts (241).

3. Plasmastrahlungsvorrichtung (220, 420, 620) nach Anspruch 1 oder 2, wobei
der erste Elektrodenabschnitt (231) in einem kontinuierlichen oder mit Unterbrechungen versehenen ringförmigen Muster angeordnet ist; und
der zweite Elektrodenabschnitt (232) in einem kontinuierlichen oder mit Unterbrechungen versehenen ringförmigen Muster um den ersten Elektrodenabschnitt (231), der in dem ringförmigen Muster angeordnet ist, angeordnet ist.

4. Plasmastrahlungsvorrichtung (20, 220, 420) nach einem der Ansprüche 1 bis 3, weiterhin aufweisend einen röhrenförmigen Abschnitt (50, 250), der das darin vorgesehene Einwirkungselement (31, 212, 312) enthält, das sich in eine vorbestimmte Richtung erstreckt,
wobei das Einwirkungselement (31, 212, 312) eine stabförmige Form aufweist und ein Endabschnitt des Einwirkungselements (31, 212, 312) als Einwirkungsabschnitt dient, das auf biologisches Gewebe einwirkt.

5. Plasmastrahlungsvorrichtung (20, 220, 420) nach Anspruch 4, wobei der Abschnitt zur Erzeugung eines elektrischen Felds (30, 230) in dem röhrenförmigen Abschnitt (50, 250) vorgesehen ist, um an einer Position angeordnet zu werden, die dem einen Endabschnitt des Einwirkungselements (31, 212, 312) entspricht.

6. Handstück (3, 203, 503, 603), aufweisend:
eine Plasmastrahlungsvorrichtung (20, 220, 520, 620) nach einem der Ansprüche 1 bis 5; und
einen Antriebsabschnitt (12) zum Antreiben des Einwirkungselements (31, 212, 535), wobei
der Antriebsabschnitt (12) ein Ultraschallschwingungsabschnitt zur Erzeugung von Ultraschallschwingungen ist; und
das Einwirkungselement (31, 212, 535) als Ergebnis der Übertragung der von dem Ultraschallschwingungsabschnitt erzeugten Ultraschallschwingung auf das Einwirkungselement schwingt, wodurch eine einschneidende Wirkung, eine abtragende Wirkung oder eine blutstillende Wirkung auf das biologische Gewebe ausgeführt wird.

7. Handstück (303), aufweisend:
eine Plasmastrahlungsvorrichtung (220) nach einem der Ansprüche 1 bis 5; und
einen Antriebsabschnitt (305) zum Antreiben des Einwirkungselements (312), wobei
der Antriebsabschnitt (305) ein Hochfrequenzstromversorgungsabschnitt ist, um Hochfrequenzstrom zu liefern; und
als Ergebnis des Hochfrequenzstroms, der von dem Hochfrequenzstromversorgungsabschnitt zugeführt wird und durch das Einwirkungselement (312) fließt, das Einwirkungselement (312) eine einschneidende Wirkung, eine abtragende Wirkung oder eine blutstillende Wirkung auf das biologische Gewebe ausführt.

8. Handstück (403), aufweisend:
eine Plasmastrahlungsvorrichtung (420) nach Anspruch 4 oder 5; und
eine Verschiebungsvorrichtung (460) zum Bewegen des Einwirkungselements (212) zwischen einer vorstehenden Position, in der das Einwirkungselement (212) aus dem röhrenförmigen Abschnitt (250) herausragt, und einer zurückgezogenen Position, in der das Ausmaß des Vorstehens des Einwirkungselements (212) geringer ist als in der vorstehenden Position.

9. Vorrichtung für chirurgische Operationen (1, 201, 301, 401, 501, 601), die ein Handstück (3, 203, 303, 403, 503, 603) gemäß einem der Ansprüche 6 bis 8 aufweist.

## Revendications

1. Dispositif d'irradiation à plasma (20, 220, 420, 520, 620) fourni dans une pièce à main (3, 203, 303, 403, 503, 603) incluant un élément agissant (31, 212, 312, 535) qui agit sur un tissu biologique, comprenant:
un canal d'écoulement de gaz (22, 222, 522) pour fournir à une partie d'extrémité distale de l'élément agissant (31, 212, 312, 535) un gaz fourni à l'extérieur de la pièce à main (3, 203, 303, 403, 503, 603); et
une section de génération de champ électrique (30, 230, 530) disposée dans le canal d'écoulement de gaz (22, 222, 522), la section de génération de champ électrique (30, 230, 530) incluant une première partie d'électrode (31, 231, 531), une seconde partie d'électrode (32, 232, 532) faisant face à la première partie d'électrode (31, 231, 531), et un élément diélectrique (40, 240, 540) ayant au moins une partie qui est située entre la première partie d'électrode (31, 231, 531) et la seconde partie d'électrode (32, 232, 532) et est disposée sur au moins l'une d'une surface de la première partie d'électrode (31, 231, 531) et d'une surface de la seconde partie d'électrode (32, 232, 532), la section de génération de champ électrique (30, 230, 530) est configurée pour générer un champ électrique dans un espace à l'intérieur du canal d'écoulement de gaz (22, 222, 522) en utilisant une différence de potentiel entre la première partie d'électrode (31, 231, 531) et la seconde partie d'électrode (32, 232, 532), produisant ainsi la décharge à plasma à basse température,
**caractérisé en ce que**
l'élément diélectrique (240) est configuré de telle sorte que la partie située entre la première partie d'électrode (231) et la seconde partie d'électrode (232) est en contact avec la surface de la première partie d'électrode (231) et la surface de la seconde partie d'électrode (232); au moins une partie de l'élément diélectrique (240) forme une partie de paroi interne du canal d'écoulement de gaz (222); et la décharge à plasma à basse température est produite le long de la partie de paroi interne.

2. Dispositif d'irradiation à plasma (220, 420, 620) selon la revendication 1, dans lequel l'élément diélectrique (240) a une première partie d'élément diélectrique (241) disposée sur une surface de la seconde partie d'électrode (232) sur un côté vers la première partie d'électrode (231) et une seconde partie d'élément diélectrique (242) disposée sur une surface de la seconde partie d'électrode (232) sur un côté opposé à la surface sur le côté vers la première partie d'électrode (231);
la seconde partie d'électrode (232) est une électrode dont le potentiel oscille de telle sorte qu'un potentiel de la première partie d'électrode (231) devient le centre de l'oscillation de potentiel; et
la seconde partie d'élément diélectrique (242) a une épaisseur (T4) supérieure à une épaisseur (T3) de la première partie d'élément diélectrique (241).

3. Dispositif d'irradiation à plasma (220, 420, 620) selon la revendication 1 ou 2, dans lequel
la première partie d'électrode (231) est disposée en un motif annulaire continu ou intermittent; et
la seconde partie d'électrode (232) est disposée en un motif annulaire continu ou intermittent autour de la première partie d'électrode (231) disposée en le motif annulaire.

4. Dispositif d'irradiation à plasma (20, 220, 420) selon l'une quelconque des revendications 1 à 3, comprenant en outre une partie tubulaire (50, 250) qui inclut l'élément agissant (31, 212, 312) prévu à l'intérieur et s'étendant dans une direction prédéterminée,
dans lequel l'élément agissant (31, 212, 312) a une forme analogue à une tige, et une partie d'extrémité de l'élément agissant (31, 212, 312) sert de partie agissante agissant sur un tissu biologique.

5. Dispositif d'irradiation à plasma (20, 220, 420) selon la revendication 4, dans lequel la section de génération de champ électrique (30, 230) est prévue dans la partie tubulaire (50, 250) pour être située à une position correspondant à la partie d'extrémité de l'élément agissant (31, 212, 312).

6. Pièce à main (3, 203, 503, 603) comprenant:
un dispositif d'irradiation à plasma (20, 220, 520, 620) selon l'une quelconque des revendications 1 à 5; et
une section d'entraînement (12) pour entraîner l'élément agissant (31, 212, 535), dans laquelle
la section d'entraînement (12) est une section de vibration ultrasonore pour générer une vibration ultrasonore; et
l'élément agissant (31, 212, 535) vibre par suite de la transmission de la vibration ultrasonore générée par la section de vibration ultrasonore à l'élément agissant, réalisant ainsi une action d'incision, une action d'ablation ou une action d'étanchement pour le tissu biologique.

7. Pièce à main (303) comprenant:
un dispositif d'irradiation à plasma (220) selon l'une quelconque des revendications 1 à 5; et
une section d'entraînement (305) pour entraîner l'élément agissant (312), dans laquelle
la section d'entraînement (305) est une section de fourniture de courant haute fréquence pour fournir un courant haute fréquence; et
par suite du courant haute fréquence fourni par la section de fourniture de courant haute fréquence circulant à travers l'élément agissant (312), l'élément agissant (312) réalise une action d'incision, une action d'ablation ou une action d'étanchement pour le tissu biologique.

8. Pièce à main (403) comprenant:
un dispositif d'irradiation à plasma (420) selon la revendication 4 ou 5; et
un dispositif de déplacement (460) pour déplacer l'élément agissant (212) entre une position en saillie dans laquelle l'élément agissant (212) fait saillie de la partie tubulaire (250) et une position rétractée dans laquelle la quantité de saillie de l'élément agissant (212) est plus petite que celle de la position en saillie.

9. Dispositif d'opération chirurgicale (1, 201, 301, 401, 501, 601) comprenant une pièce à main (3, 203, 303, 403, 503, 603) selon l'une quelconque des revendications 6 à 8.
